# EUROPEAN PATENT SPECIFICATION

(11) **EP 2 408 914 B1**
(45) Date of publication and mention of the grant of the patent: **12.06.2013**
(21) Application number: 10739694.7
(22) Date of filing: 14.07.2010
(51) Int. Cl.: C12N 15/10, C12Q 1/70

(54) **METHOD FOR DETERMINING THE SENSITIVITY OR RESISTANCE OF RETROVIRUS ISOLATES TO MOLECULES AND DIAGNOSTIC KITS**
VERFAHREN ZUR BESTIMMUNG DER EMPFINDLICHKEIT ODER RESISTENZ VON RETROVIRENISOLATEN GEGEN MOLEKÜLE UND DIAGNOSEKITS DAFÜR
PROCÉDÉ DE DÉTERMINATION DE LA SENSIBILITÉ OU DE LA RÉSISTANCE D'ISOLATS DE RÉTROVIRUS À DES MOLÉCULES ET COFFRETS DE DIAGNOSTIC

(30) Priority: 16.07.2009 US 504456; 15.07.2009 US 503174
(43) Date of publication of application: 25.01.2012
(73) Proprietor: Université d'Aix-Marseille, 13284 Marseille Cedex 07 (FR); CNRS, 75794 Paris Cedex 16 (FR)
(72) Inventor: GLUSCHANKOF, Pablo, F-13015 Marseille (FR); RAOULT, Didier, F-13008 Marseille (FR); BEN M'BAREK, Najoua, F-13124 Peyrin (FR); AUDOLY, Gilles, F-13008 Marseille (FR); PERRIN-EAST, Christelle, F-13009 Marseille (FR)
(74) Representative: Touroude-Barbot, Magali Linda
(86) International application number: PCT/IB2010/001738
(87) International publication number: WO 2011/007244

(56) References cited:
- M'BAREK NAJOUA BEN ET AL: "HIV-2 Protease resistance defined in yeast cells" RETROVIROLOGY, BIOMED CENTRAL LTD., LONDON, GB LNKD- DOI:10.1186/1742-4690-3-58, vol. 3, no. 1, 6 September 2006 (2006-09-06), page 58, XP021019168 ISSN: 1742-4690
- MAROZSAN ANDRE J ET AL: "Development of a yeast-based recombination cloning/system for the analysis of gene products from diverse human immunodeficiency virus type 1 isolates." JOURNAL OF VIROLOGICAL METHODS, vol. 111, no. 2, August 2003 (2003-08), pages 111-120, XP002525823 ISSN: 0166-0934
- WAN MIN ET AL: "Autoprocessing: An essential step for the activation of HIV-1 protease", BIOCHEMICAL JOURNAL, vol. 316, no. 2, 1996, pages 569-573, ISSN: 0264-6021

### SEQUENCE LISTING

<110> GLUSCHANKOF, PABLO
   PERRIN-EAST, CHRISTELLE
   RAOUL, DIDIER
   BEN M'BAREK, NAJOUA
   AUDOLY, GILLES
<120> METHOD FOR DETERMINING THE SENSITIVITY OR RESISTANCE OF RETROVIRUS ISOLATES TO MOLECULES, THERAPEUTIC RETROVIRAL TREATMENTS BASED ON VIRAL PROTEASE INHIBITORS AND DIAGNOSTIC KITS
<130> AMI-B-0001PCT1
<150> 12/503,174
   <151> 2009-07-15
<150> 12/504,456
   <151> 2009-07-16
<160> 58
<170> PatentIn version 3.5
<210> 1
   <211> 77
   <212> DNA
   <213> Artificial Sequence
<220>
   <223> Description of Artificial Sequence: Synthetic oligonucleotide
<400> 1
   ggatccggag acaccataca gggagccacc aacagcggcc gcagtagagc caataaaaat 60
   aatgctaaag cgagctc 77
<210> 2
   <211> 20
   <212> DNA
   <213> Artificial Sequence
<220>
   <223> Description of Artificial Sequence: Synthetic primer
<400> 2
   gaaagaagcc ccgcaacttc 20
<210> 3
   <211> 20
   <212> DNA
   <213> Artificial Sequence
<220>
   <223> Description of Artificial Sequence: Synthetic primer
<400> 3
   gggatccatg tcacttgcca 20
<210> 4
   <211> 60
   <212> DNA
   <213> Artificial sequence
<220>
   <223> Description of Artificial Sequence: Synthetic primer
<400> 4
   cgaggatccg gagacaccat acagggagcc accaacagcg gccgcgccat gcctcaattc 60
<210> 5
   <211> 54
   <212> DNA
   <213> Artificial sequence
<220>
   <223> Description of Artificial Sequence: Synthetic primer
<400> 5
   gcggagctcg ctttagcatt atttttattg gctctactgc ggccgcttaa gatt 54
<210> 6
   <211> 40
   <212> DNA
   <213> Artificial sequence
<220>
   <223> Description of Artificial Sequence: Synthetic oligonucleotide
<400> 6
   ggatccggag acaccataca gggagccacc aacagcggcc 40
<210> 7
   <211> 37
   <212> DNA
   <213> Artificial Sequence
<220>
   <223> Description of Artificial Sequence: Synthetic oligonucleotide
<400> 7
   gcagtagagc caataaaaat aatgctaaag cgagctc 37
<210> 8
   <211> 52
   <212> DNA
   <213> Artificial sequence
<220>
   <223> Description of Artificial Sequence: Synthetic primer
<400> 8
   gcggagctcg ctttagcatt atttttattg gctactgcgg ccgcttaaga tt 52
<210> 9
   <211> 303
   <212> DNA
   <213> Artificial Sequence
<220>
   <223> Description of Artificial Sequence: synthetic HIV protease sequence containing start (ATG) and stop (TAA) codons
<400> 9
   atgcctcaga tcactctttg gcagcgaccc ctcgtcacaa taaagatagg ggggcaatta      60
   aaggaagctc tattagatac aggagcagat gatacagtat tagaagaaat gaatttgcca     120
   ggaagatgga aaccaaaaat gataggggga attggaggtt ttatcaaagt aagacagtat     180
   gatcagatac tcatagaaat ctgcggacat aaagctatag gtacagtatt agtaggacct     240
   acacctgtca acataattgg aagaaatctg ttgactcaga ttggctgcac tttaaatttt     300
   taa                                                                   303
<210> 10
   <211> 324
   <212> DNA
   <213> Artificial Sequence
<220>
   <223> Description of Artificial sequence: synthetic HIV-1 protease sequence with 24 more nucleic acids at its 5' end, 3 more nucleic acids at its 3' end
<400> 10
   ggatccatgg tatcctttaa cttccctcag atcactcttt ggcagcgacc cctcgtcaca      60
   ataaagatag gggggcaatt aaaggaagct ctattagata caggagcaga tgatacagta     120
   ttagaagaaa tgaatttgcc aggaagatgg aaaccaaaaa tgataggggg aattggaggt     180
   tttatcaaag taagacagta tgatcagata ctcatagaaa tctgcggaca taaagctata     240
   ggtacagtat tagtaggacc tacacctgtc aacataattg gaagaaatct gttgactcag     300
   attggctgca ctttaaattt tccc                                            324
<210> 11
   <211> 381
   <212> DNA
   <213> Artificial sequence
<220>
   <223> Description of Artificial sequence: synthetic HIV-1 protease sequence with 24 more nucleic acids at its 5' end, 60 more nucleic acids in its 3' end
<400> 11
   ggatccatgg tatcctttaa cttgcctcag atcactcttt ggcagcgacc cctcgtcaca      60
   ataaagatag gggggcaatt aaaggaagct ctattagata caggagcaga tgatacagta     120
   ttagaagaaa tgaatttgcc aggaagatgg aaaccaaaaa tgataggggg aattggaggt     180
   tttatcaaag taagacagta tgatcagata ctcatagaaa tctgcggaca taaagctata     240
   ggtacagtat tagtaggacc tacacctgtc aacataattg gaagaaatct gttgactcag     300
   attggctgca ctttaaattt tcccattagt ccaattgaaa ctgtaccagt aaaattaaag     360
   ccaggaatgg attaagagct c                                               381
<210> 12
   <211> 327
   <212> DNA
   <213> Artificial Sequence
<220>
   <223> Description of Artificial Sequence: synthetic HIV-1 protease sequence with 27 more nucleic acids at its 5' end, 3 more nucleic acids in its 3' end
<400> 12
   ggatccatga ctgtatcctt taacttccct cagatcactc tttggcagcg acccctcgtc      60
   acaataaaga taggggggca attaaaggaa gctctattag atacaggagc agatgataca     120
   gtattagaag aaatgaattt gccaggaaga tggaaaccaa aaatgatagg gggaattgga     180
   ggttttatca aagtaagaca gtatgatcag atactcatag aaatctgcgg acataaagct     240
   ataggtacag tattagtagg acctacacct gtcaacataa ttggaagaaa tctgttgact     300
   cagattggct gcactttaaa ttttccc                                         327
<210> 13
   <211> 384
   <212> DNA
   <213> Artificial Sequence
<220>
   <223> Description of Artificial sequence: synthetic HIV-1 protease sequence with 27 more nucleic acids at its 5' end, 60 more nucleic acids in its 3' end
<400> 13
   ggatccatga ctgtatcctt taacttgcct cagatcactc tttggcagcg acccctcgtc      60
   acaataaaga taggggggca attaaaggaa gctctattag atacaggagc agatgataca     120
   gtattagaag aaatgaattt gccaggaaga tggaaaccaa aaatgatagg gggaattgga     180
   ggttttatca aagtaagaca gtatgatcag atactcatag aaatctgcgg acataaagct     240
   ataggtacag tattagtagg acctacacct gtcaacataa ttggaagaaa tctgttgact     300
   cagattggct gcactttaaa ttttcccatt agtccaattg aaactgtacc agtaaaatta     360
   aagccaggaa tggattaaga gctc                                            384
<210> 14
   <211> 387
   <212> DNA
   <213> Artificial Sequence
<220>
   <223> Description of Artificial sequence: synthetic HIV-1 protease sequence with 30 more nucleic acids at its 5' end, 60 more in its 3' end
<400> 14
   ggatccatgg gaactgtatc ctttaacttg cctcagatca ctctttggca gcgacccctc      60
   gtcacaataa agataggggg gcaattaaag gaagctctat tagatacagg agcagatgat     120
   acagtattag aagaaatgaa tttgccagga agatggaaac caaaaatgat agggggaatt     180
   ggaggtttta tcaaagtaag acagtatgat cagatactca tagaaatctg cggacataaa     240
   gctataggta cagtattagt aggacctaca cctgtcaaca taattggaag aaatctgttg     300
   actcagattg gctgcacttt aaattttccc attagtccaa ttgaaactgt accagtaaaa     360
   ttaaagccag gaatggatta agagctc                                         387
<210> 15
   <211> 435
   <212> DNA
   <213> Artificial sequence
<220>
   <223> Description of Artificial sequence: Synthetic HIV-1 short precursor sequence
<400> 15
   ggatccatgt ggggtagaga caacaactcc ctctcagaag caggagccga tagacaagga      60
   actgtatcct ttagcttccc tcagatcact ctttggcagc gacccctcgt cacaataaag     120
   ataggggggc aattaaagga agctctatta gatacaggag cagatgatac agtattagaa      180
   gaaatgaatt tgccaggaag atggaaacca aaaatgatag ggggaattgg aggttttatc     240
   aaagtaagac agtatgatca gatactcata gaaatctgcg gacataaagc tataggtaca     300
   gtattagtag gacctacacc tgtcaacata attggaagaa atctgttgac tcagattggc     360
   tgcactttaa attttcccat tagtccaatt gaaactgtac cagtaaaatt aaagccagga     420
   atggattaag agctc                                                      435
<210> 16
   <211> 435
   <212> DNA
   <213> Artificial Sequence
<220>
   <223> Description of Artificial sequence: Synthetic HIV-1 short precursor sequence with cleavage site mutation downstream the protease coding sequence
<400> 16
   ggatccatgt ggggtagaga caacaactcc ctctcagaag caggagccga tagacaagga    60
   actgtatcct ttagcttccc tcagatcact ctttggcagc gacccctcgt cacaataaag   120
   ataggggggc aattaaagga agctctatta gatacaggag cagatgatac agtattagaa   180
   gaaatgaatt tgccaggaag atggaaacca aaaatgatag ggggaattgg aggttttatc   240
   aaagtaagac agtatgatca gatactcata gaaatctgcg gacataaagc tataggtaca   300
   gtattagtag gacctacacc tgtcaacata attggaagaa atctgttgac tcagattggc   360
   tgcactttaa atattcccat tagtccaatt gaaactgtac cagtaaaatt aaagccagga   420
   atggattaag agctc                                                    435
<210> 17
   <211> 792
   <212> DNA
   <213> Artificial Sequence
<220>
   <223> Description of Artificial Sequence: synthetic HIV-1 long precursor sequence with modification of the open reading frame
<400> 17
   ggatccatga tgacagcatg tcagggagtg gggggacccg gccataaagc aagagttttg    60
   gctgaagcaa tgagccaagt aacaaatcca gctaccataa tgatacagaa aggcaatttt   120
   aggaaccaaa gaaagactgt taagtgtttc aattgtggca aagaagggca catagccaaa   180
   aattgcaggg cccctaggaa aaagggctgt tggaaatgtg gaaaggaagg acaccaaatg   240
   aaagattgta ctgagagaca ggctaatttt ttaagggaag atctggcctt cccacaaggg   300
   aaggccaggg aattttcttc agagcagacc agagccaaca gccccaccag aagagagctt   360
   caggtttggg gtagagacaa caactccctc tcagaagcag gagccgatag acaaggaact   420
   gtatccttta acttccctca gatcactctt tggcagcgac ccctcgtcac aataaagata   480
   ggggggcaat taaaggaagc tctattagat acaggagcag atgatacagt attagaagaa   540
   atgaatttgc caggaagatg gaaaccaaaa atgatagggg gaattggagg ttttatcaaa   600
   gtaagacagt atgatcagat actcatagaa atctgcggac ataaagctat aggtacagta   660
   ttagtaggac ctacacctgt caacataatt ggaagaaatc tgttgactca gattggctgc   720
   actttaaatt ttcccattag tccaattgaa actgtaccag taaaattaaa gccaggaatg   780
   gattaagagc tc                                                       792
<210> 18
   <211> 4345
   <212> DNA
   <213> Artificial Sequence
<220>
   <223> Description of Artificial Sequence: synthetic HIV-1 Gag -pol sequence
<400> 18
   gcggagtcta gaaggagaga gatgggtgcg agagcgtcgg tattaagcgg gggagaatta      60
   gataaatggg aaaaaattcg gttaaggcca gggggaaaga aacaatataa actaaaacat     120
   atagtatggg caagcaggga gctagaacga ttcgcagtta atcctggcct tttagagaca     180
   tcagaaggct gtagacaaat actgggacag ctacaaccat cccttcagac aggatcagaa     240
   gaacttagat cattatataa tacaatagca gtcctctatt gtgtgcatca aaggatagat     300
   gtaaaagaca ccaaggaagc cttagataag atagaggaag agcaaaacaa aagtaagaaa     360
   aaggcacagc aagcagcagc tgacacagga aacaacagcc aggtcagcca aaattaccct     420
   atagtgcaga acctccaggg gcaaatggta catcaggcca tatcacctag aactttaaat     480
   gcatgggtaa aagtagtaga agagaaggct ttcagcccag aagtaatacc catgttttca     540
   gcattatcag aaggagccac cccacaagat ttaaatacca tgctaaacac agtgggggga     600
   catcaagcag ccatgcaaat gttaaaagag accatcaatg aggaagctgc agaatgggat     660
   agattgcatc cagtgcatgc agggcctatt gcaccaggcc agatgagaga accaagggga     720
   agtgacatag caggaactac tagtaccctt caggaacaaa taggatggat gacacataat     780
   ccacctatcc cagtaggaga aatctataaa agatggataa tcctgggatt aaataaaata     840
   gtaagaatgt atagccctac cagcattctg gacataagac aaggaccaaa ggaacccttt     900
   agagactatg tagaccgatt ctataaaact ctaagagccg agcaagcttc acaagaggta     960
   aaaaattgga tgacagaaac cttgttggtc caaaatgcga acccagattg taagactatt 1020
   ttaaaagcat tgggaccagg agcgacacta gaagaaatga tgacagcatg tcagggagtg 1080
   gggggacccg gccataaagc aagagttttg gctgaagcaa tgagccaagt aacaaatcca 1140
   gctaccataa tgatacagaa aggcaatttt aggaaccaaa gaaagactgt taagtgtttc 1200
   aattgtggca aagaagggca catagccaaa aattgcaggg cccctaggaa aaagggctgt 1260
   tggaaatgtg gaaaggaagg acaccaaatg aaagattgta ctgagagaca ggctaatttt 1320
   ttagggaaga tctggccttc ccacaaggga aggccaggga attttcttca gagcagacca 1380
   gagccaacag ccccaccaga agagagcttc aggtttgggg aagagacaac aactccctct 1440
   cagaagcagg agccgataga caaggaactg tatcctttag cttccctcag atcactcttt 1500
   ggcagcgacc cctcgtcaca ataaagatag gggggcaatt aaaggaagct ctattagata 1560
   caggagcaga tgatacagta ttagaagaaa tgaatttgcc aggaagatgg aaaccaaaaa 1620
   tgataggggg aattggaggt tttatcaaag taagacagta tgatcagata ctcatagaaa 1680
   tctgcggaca taaagctata ggtacagtat tagtaggacc tacacctgtc aacataattg 1740
   gaagaaatct gttgactcag attggctgca ctttaaattt tcccattagt cctattgaga 1800
   ctgtaccagt aaaattaaag ccaggaatgg atggcccaaa agttaaacaa tggccattga 1860
   cagaagaaaa aataaaagca ttagtagaaa tttgtacaga aatggaaaag gaaggaaaaa 1920
   tttcaaaaat tgggcctgaa aatccataca atactccagt atttgccata aagaaaaaag 1980
   acagtactaa atggagaaaa ttagtagatt tcagagaact taataagaga actcaagatt 2040
   tctgggaagt tcaattagga ataccacatc ctgcagggtt aaaacagaaa aaatcagtaa 2100
   cagtactgga tgtgggcgat gcatattttt cagttccctt agataaagac ttcaggaagt 2160
   atactgcatt taccatacct agtataaaca atgagacacc agggattaga tatcagtaca 2220
   atgtgcttcc acagggatgg aaaggatcac cagcaatatt ccagtgtagc atgacaaaaa 2280
   tcttagagcc ttttagaaaa caaaatccag acatagtcat ctatcaatac atggatgatt 2340
   tgtatgtagg atctgactta gaaatagggc agcatagaac aaaaatagag gaactgagac 2400
   aacatctgtt gaggtgggga tttaccacac cagacaaaaa acatcagaaa gaacctccat 2460
   tcctttggat gggttatgaa ctccatcctg ataaatggac agtacagcct atagtgctgc 2520
   cagaaaagga cagctggact gtcaatgaca tacagaaatt agtgggaaaa ttgaattggg 2580
   caagtcagat ttatgcaggg attaaagtaa ggcaattatg taaacttctt aggggaacca 2640
   aagcactaac agaagtagta ccactaacag aagaagcaga gctagaactg gcagaaaaca 2700
   gggagattct aaaagaaccg gtacatggag tgtattatga cccatcaaaa gacttaatag 2760
   cagaaataca gaagcagggg caaggccaat ggacatatca aatttatcaa gagccattta 2820
   aaaatctgaa aacaggaaaa tatgcaagaa tgaagggtgc ccacactaat gatgtgaaac 2880
   aattaacaga ggcagtacaa aaaatagcca cagaaagcat agtaatatgg ggaaagactc 2940
   ctaaatttaa attacccata caaaaggaaa catgggaagc atggtggaca gagtattggc 3000
   aagccacctg gattcctgag tgggagtttg tcaatacccc tcccttagtg aagttatggt    3060
   accagttaga gaaagaaccc ataataggag cagaaacttt ctatgtagat ggggcagcca    3120
   atagggaaac taaattagga aaagcaggat atgtaactga cagaggaaga caaaaagttg    3180
   tccccctaac ggacacaaca aatcagaaga ctgagttaca agcaattcat ctagctttgc    3240
   aggattcggg attagaagta aacatagtga cagactcaca atatgcattg ggaatcattc    3300
   aagcacaacc agataagagt gaatcagagt tagtcagtca aataatagag cagttaataa    3360
   aaaaggaaaa agtctacctg gcatgggtac cagcacacaa aggaattgga ggaaatgaac    3420
   aagtagatgg gttggtcagt gctggaatca ggaaagtact atttttagat ggaatagata    3480
   aggcccaaga agaacatgag aaatatcaca gtaattggag agcaatggct agtgatttta    3540
   acctaccacc tgtagtagca aaagaaatag tagccagctg tgataaatgt cagctaaaag    3600
   gggaagccat gcatggacaa gtagactgta gcccaggaat atggcagcta gattgtacac    3660
   atttagaagg aaaagttatc ttggtagcag ttcatgtagc cagtggatat atagaagcag    3720
   aagtaattcc agcagagaca gggcaagaaa cagcatactt cctcttaaaa ttagcaggaa    3780
   gatggccagt aaaaacagta catacagaca atggcagcaa tttcaccagt actacagtta    3840
   aggccgcctg ttggtgggcg gggatcaagc aggaatttgg cattccctac aatccccaaa    3900
   gtcaaggagt aatagaatct atgaataaag aattaaagaa aattatagga caggtaagag    3960
   atcaggctga acatcttaag acagcagtac aaatggcagt attcatccac aattttaaaa    4020
   gaaaaggggg gattgggggg tacagtgcag gggaaagaat agtagacata atagcaacag    4080
   acatacaaac taaagaatta caaaaacaaa ttacaaaaat tcaaaatttt cgggtttatt    4140
   acagggacag cagagatcca gtttggaaag gaccagcaaa gctcctctgg aaaggtgaag    4200
   gggcagtagt aatacaagat aatagtgaca taaaagtagt gccaagaaga aaagcaaaga    4260
   tcatcaggga ttatggaaaa cagatggcag gtgatgattg tgtggcaagt agacaggatg    4320
   aggattaaca ctcgagaaag attag                                          4345
<210> 19
   <211> 326
   <212> DNA
   <213> Artificial Sequence
<220>
   <223> Description of Artificial sequence: synthetic HIV-1 protease sequence with a start codon at position -1 and a stop codon after 3 more nucleic acids
<400> 19
   ggatccttta acatgcctca gatcactctt tggcagcgac ccctcgtcac aataaagata 60
   ggggggcaat taaaggaagc tctattagat acaggagcag atgatacagt attagaagaa 120
   atgaatttgc caggaagatg gaaaccaaaa atgatagggg gaattggagg ttttatcaaa 180
   gtaagacagt atgatcagat actcatagaa atctgcggac ataaagctat aggtacagta 240
   ttagtaggac ctacacctgt caacataatt ggaagaaatc tgttgactca gattggctgc 300
   actttaaatt ttccctaagc tccaat 326
<210> 20
   <211> 329
   <212> DNA
   <213> Artificial Sequence
<220>
   <223> Description of Artificial Sequence: synthetic HIV-1 protease sequence with a start codon at position -1 and a stop codon after 6 more nucleic acids
<400> 20
   ggatccttta acatgcctca gatcactctt tggcagcgac ccctcgtcac aataaagata 60
   ggggggcaat taaaggaagc tctattagat acaggagcag atgatacagt attagaagaa 120
   atgaatttgc caggaagatg gaaaccaaaa atgatagggg gaattggagg ttttatcaaa 180
   gtaagacagt atgatcagat actcatagaa atctgcggac ataaagctat aggtacagta 240
   ttagtaggac ctacacctgt caacataatt ggaagaaatc tgttgactca gattggctgc 300
   actttaaatt ttcccattta agctccaat 329
<210> 21
   <211> 332
   <212> DNA
   <213> Artificial Sequence
<220>
   <223> Description of Artificial Sequence: Synthetic HIV-1 protease sequence with a start codon at position -1 and a stop codon after 12 more nucleic acids
<400> 21
   ggatccttta acatgcctca gatcactctt tggcagcgac ccctcgtcac aataaagata 60
   ggggggcaat taaaggaagc tctattagat acaggagcag atgatacagt attagaagaa 120
   atgaatttgc caggaagatg gaaaccaaaa atgatagggg gaattggagg ttttatcaaa 180
   gtaagacagt atgatcagat actcatagaa atctgcggac ataaagctat aggtacagta 240
   ttagtaggac ctacacctgt caacataatt ggaagaaatc tgttgactca gattggctgc 300
   actttaaatt ttcccattag ctaagctcca at 332
<210> 22
   <211> 340
   <212> DNA
   <213> Artificial Sequence
<220>
   <223> Description of Artificial sequence: Synthetic HIV-1 protease sequence with a start codon at position -1 and a stop codon after 16 more nucleic acids
<400> 22
   ggatccttta acatgcctca gatcactctt tggcagcgac ccctcgtcac aataaagata      60
   ggggggcaat taaaggaagc tctattagat acaggagcag atgatacagt attagaagaa    120
   atgaatttgc caggaagatg gaaaccaaaa atgatagggg gaattggagg ttttatcaaa    180
   gtaagacagt atgatcagat actcatagaa atctgcggac ataaagctat aggtacagta    240
   ttagtaggac ctacacctgt caacataatt ggaagaaatc tgttgactca gattggctgc    300
   actttaaatt ttcccattag tcctaatagg taataggtaa                          340
<210> 23
   <211> 371
   <212> DNA
   <213> Artificial sequence
<220>
   <223> Description of Artificial Sequence: synthetic HIV-1 protease sequence with a start codon at position -1 and a stop codon after 51 more nucleic acids
<400> 23
   ggatccttta acatgcctca gatcactctt tggcagcgac ccctcgtcac aataaagata      60
   ggggggcaat taaaggaagc tctattagat acaggagcag atgatacagt attagaagaa    120
   atgaatttgc caggaagatg gaaaccaaaa atgatagggg gaattggagg ttttatcaaa    180
   gtaagacagt atgatcagat actcatagaa atctgcggac ataaagctat aggtacagta    240
   ttagtaggac ctacacctgt caacataatt ggaagaaatc tgttgactca gattggctgc    300
   actttaaatt ttcccattag tccaattgaa actgtaccag taaaattaaa gccaggaatg    360
   gattaagagc t                                                         371
<210> 24
   <211> 388
   <212> DNA
   <213> Artificial Sequence
<220>
   <223> Description of Artificial sequence: synthetic HIV-1 protease sequence with a start codon at position -1 and a stop codon after 66 more nucleic acids
<400> 24
   ggatccttta acatgcctca gatcactctt tggcagcgac ccctcgtcac aataaagata      60
   ggggggcaat taaaggaagc tctattagat acaggagcag atgatacagt attagaagaa    120
   atgaatttgc caggaagatg gaaaccaaaa atgatagggg gaattggagg ttttatcaaa    180
   gtaagacagt atgatcagat actcatagaa atctgcggac ataaagctat aggtacagta    240
   ttagtaggac ctacacctgt caacataatt ggaagaaatc tgttgactca gattggctgc    300
   actttaaatt ttcccattag tcctattgag actgtaccag taaaattaaa gccaggaatg    360
   gatggcccaa aagttaaata ataggtaa                                        388
<210> 25
   <211> 477
   <212> DNA
   <213> Artificial Sequence
<220>
   <223> Description of Artificial sequence: Synthetic HIV-1 protease sequence with a start codon at position -1 and a stop codon after 156 more nucleic acids
<400> 25
   ggatccttta acatgcctca gatcactctt tggcagcgac ccctcgtcac aataaagata      60
   ggggggcaat taaaggaagc tctattagat acaggagcag atgatacagt attagaagaa    120
   atgaatttgc caggaagatg gaaaccaaaa atgatagggg gaattggagg ttttatcaaa    180
   gtaagacagt atgatcagat actcatagaa atctgcggac ataaagctat aggtacagta    240
   ttagtaggac ctacacctgt caacataatt ggaagaaatc tgttgactca gattggctgc    300
   actttaaatt ttcccattag tcctattgag actgtaccag taaaattaaa gccaggaatg    360
   gatggcccaa aagttaaaca atggccattg acagaagaaa aaataaaagc attagtagaa    420
   atttgtacag aaatggaaaa ggaaggaaaa atttcaaaaa ttgggcctaa taggtaa        477
<210> 26
   <211> 669
   <212> DNA
   <213> Artificial Sequence
<220>
   <223> Description of Artificial sequence: Synthetic HIV-1 protease sequence with a start codon at position -1 and a stop codon after 342 more nucleic acids
<400> 26
   ggatccttta acatgcctca gatcactctt tggcagcgac ccctcgtcac aataaagata 60
   ggggggcaat taaaggaagc tctattagat acaggagcag atgatacagt attagaagaa 120
   atgaatttgc caggaagatg gaaaccaaaa atgatagggg gaattggagg ttttatcaaa 180
   gtaagacagt atgatcagat actcatagaa atctgcggac ataaagctat aggtacagta 240
   ttagtaggac ctacacctgt caacataatt ggaagaaatc tgttgactca gattggctgc 300
   actttaaatt tttcctatta gtcctattga gactgtacca gtaaaattaa agccaggaat 360
   ggatggccca aaagttaaac aatggccatt gacagaagaa aaaataaaag cattagtaga 420
   aatttgtaca gaaatggaaa aggaaggaaa aatttcaaaa attgggcctg aaaatccata 480
   caatactcca gtatttgcca taaagaaaaa agacagtact aaatggagaa aattagtaga 540
   tttcagagaa cttaataaga gaactcaaga tttctgggaa gttcaattag gaataccaca 600
   tcctgcaggg ttaaaacaga aaaaatcagt aacagtactg gatgtgggcg atgctaatag 660
   gtaataggt 669
<210> 27
   <211> 796
   <212> DNA
   <213> Artificial Sequence
<220>
   <223> Description of Artificial Sequence: Synthetic HIV-1 protease sequence with a start codon at position -1 and a stop codon after 471 more nucleic acids
<400> 27
   ggatccttta acatgcctca gatcactctt tggcagcgac ccctcgtcac aataaagata 60
   ggggggcaat taaaggaagc tctattagat acaggagcag atgatacagt attagaagaa 120
   atgaatttgc caggaagatg gaaaccaaaa atgatagggg gaattggagg ttttatcaaa 180
   gtaagacagt atgatcagat actcatagaa atctgcggac ataaagctat aggtacagta 240
   ttagtaggac ctacacctgt caacataatt ggaagaaatc tgttgactca gattggctgc 300
   actttaaatt ttcccattag tcctattgag actgtaccag taaaattaaa gccaggaatg 360
   gatggcccaa aagttaaaca atggccattg acatgaagaa aaaataaaag cattagtaga 420
   aatttgtaca gaaatggaaa aggaaggaaa aatttcaaaa attgggcctg aaaatccata 480
   caatactcca gtatttgcca taaagaaaaa agacagtact aaatggagaa aattagtaga 540
   tttcagagaa cttaataaga gaactcaaga tttctgggaa gttcaattag gaataccaca 600
   tcctgcaggg ttaaaacaga aaaaatcagt aacagtactg gatgtgggcg atgcatattt 660
   ttcagttccc ttagataaag acttcaggaa gtatactgca tttaccatac ctagtataaa 720
   caatgagaca ccagggatta gatatcagta caatgtgctt ccacagggat ggaaaggatc 780
   actaataggt aatagg 796
<210> 28
   <211> 922
   <212> DNA
   <213> Artificial Sequence
<220>
   <223> Description of Artificial Sequence: Synthetic HIV-1 protease sequence with a start codon at position -1 and a stop codon after 591 more nucleic acids
<400> 28
   ggatccttta acatgcctca gatcactctt tggcagcgac ccctcgtcac aataaagata      60
   ggggggcaat taaaggaagc tctattagat acaggagcag atgatacagt attagaagaa    120
   atgaatttgc caggaagatg gaaaccaaaa atgatagggg gaattggagg ttttatcaaa    180
   gtaagacagt atgatcagat actcatagaa atctgcggac ataaagctat aggtacagta    240
   ttagtaggac ctacacctgt caacataatt ggaagaaatc tgttgactca gattggctgc    300
   actttaaatt ttcccattag tcctattgag actgtaccag taaaattaaa gccaggaatg    360
   gatggcccaa aagttaaaca atggccattg acagaagaaa aaataaaagc attagtagaa    420
   atttgtacag aaatggaaaa ggaaggaaaa atttcaaaaa ttgggcctga aaatccatac    480
   aatactccag tatttgccat aaagaaaaaa gacagtacta aatggagaaa attagtagat    540
   ttcagagaac ttaataagag aactcaagat ttctgggaag ktcaattarg aataccacat    600
   cctgcagggt taaaacagaa aaaatcagta acagtactgg atgtgggcga tgcatatttt    660
   tcagttccct tagataaaga cttcaggaag tatactgcat ttaccatacc tagtataaac    720
   aatgagacac cagggattag atatcagtac aatgtgcttc cacagggatg gaaaggatca    780
   ccagcaatat tccagtgtag catgacaaaa atcttagagc cttttagaaa acaaaatcca    840
   gacatagtca tctatcaata catggatgat ttgtatgtag gatctgactt agaaataggg    900
   ctaataggta ataggtaaga gc                                              922
<210> 29
   <211> 994
   <212> DNA
   <213> Artificial sequence
<220>
   <223> Description of Artificial sequence: Synthetic HIV-1 protease sequence with a start codon at position -1 and a stop codon after 663 more nucleic acids
<400> 29
   ggatccttta acatgcctca gatcactctt tggcagcgac ccctcgtcac aataaagata      60
   ggggggcaat taaaggaagc tctattagat acaggagcag atgatacagt attagaagaa     120
   atgaatttgc caggaagatg gaaaccaaaa atgatagggg gaattggagg ttttatcaaa     180
   gtaagacagt atgatcagat actcatagaa atctgcggac ataaagctat aggtacagta     240
   ttagtaggac ctacacctgt caacataatt ggaagaaatc tgttgactca gattggctgc     300
   actttaaatt ttcccattag tcctattgag actgtaccag taaaattaaa gccaggaatg     360
   gatggcccaa aagttaaaca atggccattg acagaagaaa aaataaaagc attagtagaa     420
   atttgtacag aaatggaaaa ggaaggaaaa atttcaaaaa ttgggcctga aaatccatac     480
   aatactccag tatttgccat aaagaaaaaa gacagtacta aatggagaaa attagtagat     540
   ttcagagaac ttaataagag aactcaagat ttctgggaag ttcaattagg aataccacat     600
   cctgcagggt taaaacagaa aaaatcagta acagtactgg atgtgggcga tgcatatttt     660
   tcagttccct tagataaaga cttcaggaag tatactgcat ttaccatacc tagtataaac     720
   aatgagacac cagggattag atatcagtac aatgtgcttc cacagggatg gaaaggatca     780
   ccagcaatat tccagtgtag catgacaaaa atcttagagc cttttagaaa acaaaatcca     840
   gacatagtca tctatcaata catggatgat ttgtatgtag gatctgactt agaaataggg     900
   cagcatagaa caaaaataga ggaactgrga caacatctgt tgaggtgggg atttaccaca     960
   ccagacaaaa aactaatagg taataggtaa gagc                                 994
<210> 30
   <211> 1002
   <212> DNA
   <213> Artificial Sequence
<220>
   <223> Description of Artificial Sequence: Synthetic HIV-1 protease sequence with a start codon at position -1 and a stop codon after 675 more nucleic acids
<400> 30
   ggatccttta acatgcctca gatcactctt tggcagcgac ccctcgtcac aataaagata      60
   ggggggcaat taaaggaagc tctattagat acaggagcag atgatacagt attagaagaa     120
   atgaatttgc caggaagatg gaaaccaaaa atgatagggg gaattggagg ttttatcaaa     180
   gtaagacagt atgatcagat actcatagaa atctgcggac ataaagctat aggtacagta     240
   ttagtaggac ctacacctgt caacataatt ggaagaaatc tgttgactca gattggctgc    300
   actttaaatt ttcccattag tcctattgag actgtaccag twaaattaaa gccaggaatg    360
   gatggcccaa aagttaaaca atggccattg acagaagaaa aaataaaagc attagtagaa    420
   atttgtacag aaatggaaaa ggaaggaaaa atttcaaaaa ttgggcctga aaatccatac    480
   aatactccag tatttgccat aaagaaaaaa gacagtacta aatggagaaa attagtagat    540
   ttcagagaac ttaataagag aactcaagat ttctgggaag ttcaattagg aataccacat    600
   cctgcagggt taaaacagaa aaaatcagta acagtactgg atgtgggcga tgcatatttt    660
   tcagttccct tagataaaga cttcaggaag tatactgcat ttaccatacc tagtataaac    720
   aatgagacac cagggattag atatcagtac aatgtgcttc cacagggatg gaaaggatca    780
   ccagcaatat tccagtgtag catgacaaaa atcttagagc cttttagaaa acaaaatcca    840
   gacatagtca tctatcaata catggatgat ttgtatgtag gatctgactt agaaataggg    900
   cagcatagaa caaaaataga ggaactgaga caacatctgt tgaggtgggg atttaccaca    960
   ccagacaaaa aacatcagaa agaacctaat aggtaatagg ta                      1002
<210> 31
   <211> 1005
   <212> DNA
   <213> Artificial Sequence
<220>
   <223> Description of Artificial Sequence: Synthetic HIV-1 protease sequence with a start codon at position -1 and a stop codon after 678 more nucleic acids
<400> 31
   ggatccttta acatgcctca gatcactctt tggcagcgac ccctcgtcac aataaagata 60
   ggggggcaat taaaggaagc tctattagat acaggagcag atgatacagt attagaagaa 120
   atgaatttgc caggaagatg gaaaccaaaa atgatagggg gaattggagg ttttatcaaa 180
   gtaagacagt atgatcagat actcatagaa atctgcggac ataaagctat aggtacagta 240
   ttagtaggac ctacacctgt caacataatt ggaagaaatc tgttgactca gattggctgc 300
   actttaaatt ttcmcattat tcctattgas actgtaccag tcaaattaaa gccmggagtg 360
   gatggcccaa aagttaaaca atggccattg acagaagaaa aaataaaagc attagtagaa 420
   atttgtacag aaatggaaaa ggaaggaaaa atttcaaaaa ttgggcctga aaatccatac 480
   aatactccag tatttgccat aaagaaaaaa gacagtacta aatggagaaa attagtagat 540
   ttcagagaac ttaataagag aactcaagat ttctgggaag ttcaattagg aataccacat 600
   cctgcagggt taaaacagaa aaaatcagta acagtactgg atgtgggcga tgcatatttt 660
   tcagttccct tagataaaga cttcaggaag tatactgcat ttaccatacc tagtataaac 720
   aatgagacac cagggattag atatcagtac aatgtgcttc cacagggatg gaaaggatca 780
   ccagcaatat tccagtgtag catgacaaaa atcttagagc cttttagaaa acaaaatcca 840
   gacatagtca tctatcaata catggatgat ttgtatgtag gatctgactt agaaataggg 900
   cagcatagaa caaaaataga ggaactgaga caacatctgt tgaggtgggg atttaccaca 960
   ccagacaaaa aacatcagaa agaacctcct aataggtaat aggta 1005
<210> 32
   <211> 1015
   <212> DNA
   <213> Artificial Sequence
<220>
   <223> Description of Artificial Sequence: Synthetic HIV-1 protease sequence with a start codon at position -1 and a stop codon after 684 more nucleic acids
<400> 32
   ggatccttta acatgcctca gatcactctt tggcagcgac ccctcgtcac aataaagata 60
   ggggggcaat taaaggaagc tctattagat acaggagcag atgatacagt attagaagaa 120
   atgaatttgc caggaagatg gaaaccaaaa atgatagggg gaattggagg ttttatcaaa 180
   gtaagacagt atgatcagat actcatagaa atctgcggac ataaagctat aggtacagta 240
   ttagtaggac ctacacctgt caacataatt ggaagaaatc tgttgactca gattggctgc 300
   actttaaatt ttcccattag tcctattgag actgtaccag taaaattaaa gccaggaatg 360
   gatggcccaa aagttaaaca atggccattg acagaagaaa aaataaaagc attagtagaa 420
   atttgtacag aaatggaaaa ggaaggaaaa atttcaaaaa ttgggcctga aaatccatac 480
   aatactccag tatttgccat aaagaaaaaa gacagtacta aatggagaaa attagtagat 540
   ttcagagaac ttaataagag aactcaagat ttctgggaag ttcaattagg aataccacat 600
   cctgcagggt taaaacagaa aaaatcagta acagtactgg atgtgggcga tgcatatttt 660
   tcagttccct tagataaaga cttcaggaag tatactgcat ttaccatacc tagtataaac 720
   aatgagacac cagggattag atatcagtac aatgtgcttc cacagggatg gaaaggatca 780
   ccagcaatat tccagtgtag catgacaaaa atcttagagc cttttagaaa acaaaatcca 840
   gacatagtca tctatcaata catggatgat ttgtatgtag gatctgactt agaaataggg 900
   cagcatagaa caaaaataga ggractgaga caacatctgt tgaggtgggg atttaccaca 960
   ccagacaaaa aacatcagaa agaacctcca ttcctaatag gtaataggta agagc 1015
<210> 33
   <211> 1257
   <212> DNA
   <213> Artificial Sequence
<220>
   <223> Description of Artificial sequence: Synthetic HIV-1 protease sequence with a start codon at position -1 and a stop codon after 939 more nucleic acids
<400> 33
   ggatccttta acatgcctca gatcactctt tggcagcgac ccctcgtcac aataaagata 60
   ggggggcaat taaaggaagc tctattagat acaggagcag atgatacagt attagaagaa 120
   atgaatttgc caggaagatg gaaaccaaaa atgatagggg gaattggagg ttttatcaaa 180
   gtaagacagt atgatcagat actcatagaa atctgcggac ataaagctat aggtacagta 240
   ttagtaggac ctacacctgt caacataatt ggaagaaatc tgttgactca gattggctgc 300
   actttaaatt ttcccattag tcctattgag actgtaccag taaaattaaa gccaggaatg 360
   gatggcccaa aagttaaaca atggccattg acagaagaaa aaataaaagc attagtagaa 420
   atttgtacag aaatggaaaa ggaaggaaaa atttcaaaaa ttgggcctga aaatccatac 480
   aatactccag tatttgccat aaagaaaaaa gacagtacta aatggagaaa attagtagat 540
   ttcagagaac ttaataagag aactcaagat ttctgggaag ttcaattagg aataccacat 600
   cctgcagggt taaaacagaa aaaatcagta acagtactgg atgtgggcga tgcatatttt 660
   tcagttccct tagataaaga cttcaggaag tatactgcat ttaccatacc tagtataaac 720
   aatgagacac cagggattag atatcagtac aatgtgcttc cacagggatg gaaaggatca 780
   ccagcaatat tccagtgtag catgacaaaa atcttagagc cttttagaaa acaaaatcca 840
   gacatagtca tctatcaata catggatgat ttgtatgtag gatctgactt agaaataggg 900
   cagcatagaa caaaaataga ggaactgaga caacatctgt tgaggtgggg atttaccaca 960
   ccagacaaaa aacatcagaa agaacctcca ttcctttgga tgggttatga actccatcct 1020
   gataaatgga cagtacagcc tatagtgctg ccagaaaagg acagctggac tgtcaatgac 1080
   atacagaaat tagtgggaaa attgaattgg gcaagtcaga tttatgcagg gattaaagta 1140
   aggcaattat gtaaacttct taggggaacc aaagcactaa cagragtagt accactaaca 1200
   gaagaagcag agctagaact ggcagaaaac agggagattc taaaagaacc taatagg 1257
<210> 34
   <211> 1369
   <212> DNA
   <213> Artificial Sequence
<220>
   <223> Description of Artificial Sequence: Synthetic HIV-1 protease sequence with a start codon at position -1 and a stop codon after 1044 more nucleic acids
<400> 34
   ggatccttta acatgcctca gatcactctt tggcagcgac ccctcgtcac aataaagata 60
   ggggggcaat taaaggaagc tctattagat acaggagcag atgatacagt attagaagaa 120
   atgaatttgc caggaagatg gaaaccaaaa atgatagggg gaattggagg ttttatcaaa 180
   gtaagacagt atgatcagat actcatagaa atctgcggac ataaagctat aggtacagta 240
   ttagtaggac ctacacctgt caacataatt ggaagaaatc tgttgactca gattggctgc 300
   actttaaatt ttcccattag tcctattgag actgtaccag taaaattaaa gccaggaatg 360
   gatggcccaa aagttaaaca atggccattg acagaagaaa aaataaaagc attagtagaa 420
   atttgtacag aaatggaaaa ggaaggaaaa atttcaaaaa ttgggcctga aaatccatac 480
   aatactccag tatttgccat aaagaaaaaa gacagtacta aatggagaaa attagtagat 540
   ttcagagaac ttaataagag aactcaagat ttctgggaag ttcaattagg aataccacat 600
   cctgcagggt taaaacagaa aaaatcagta acagtactgg atgtgggcga tgcatatttt 660
   tcagttycct tagataaaga cttcaggaag tatactgcat ttaccatacc tagtataaac 720
   aatgagacac cagggattag atatcagtac aatgtgcttc cacagggatg gaaaggatca 780
   ccagcaatat tccagtgtag catgacaaaa atcttagagc cttttagaaa acaaaatcca 840
   gacatagtca tctatcaata catggatgat ttgtatgtag gatctgactt agaaataggg 900
   cagcatagaa caaaaataga ggaactgaga caacatctgt tgaggtgggg atttaccaca 960
   ccagacaaaa aacatcagaa agaacctcca ttcctttgga tgggttatga actccatcct 1020
   gataaatgga cagtacagcc tatagtgctg ccagaaaagg acagctggac tgtcaatgac 1080
   atacagaaat tagtgggaaa attgaattgg gcaagtcaga tttatgcagg gattaaagta 1140
   aggcaattat gtaaacttct taggggaacc aaagcactaa cagaagtagt accactaaca 1200
   gaagaagcag agctagaact ggcagaaaac agggagattc taaaagaacc ggtacatgga 1260
   gtgtattatg acccatcaaa agacttaata gcagaaatac agaagcaggg gcaaggccaa 1320
   tggacatatc aaatttatca agagccatta aaaatctaat aggtaatag 1369
<210> 35
   <211> 1405
   <212> DNA
   <213> Artificial Sequence
<220>
   <223> Description of Artificial Sequence: Synthetic HIV-1 protease sequence with a start codon at position -1 and a stop codon after 1083 more nucleic acids
<400> 35
   ggatccttta acatgcctca gatcactctt tggcagcgac ccctcgtcac aataaagata 60
   ggggggcaat taaaggaagc tctattagat acaggagcag atgatacagt attagaagaa 120
   atgaatttgc caggaagatg gaaaccaaaa atgatagggg gaattggagg ttttatcaaa 180
   gtaagacagt atgatcagat actcatagaa atctgcggac ataaagctat aggtacagta 240
   ttagtaggac ctacacctgt caacataatt ggaagaaatc tgttgactca gattggctgc 300
   actttaaatt ttcccattag tcctattgag actgtaccag taaaattaaa gccaggaatg 360
   gatggcccaa aagttaaaca atggccattg acagaagaaa aaataaaagc attagtagaa 420
   atttgtacag aaatggaaaa ggaaggaaaa atttcaaaaa ttgggcctga aaatccatac 480
   aatactccag tatttgccat aaagaaaaaa gacagtacta aatggagaaa attagtagat 540
   ttcagagaac ttaataagag aactcaagat ttctgggaag ttcaattagg aataccacat     600
   cctgcagggt taaaacagaa aaaatcagta acagtactgg atgtgggcga tgcatatttt     660
   tcagttycct tagataaaga cttcaggaag tatactgcat ttaccatacc tagtataaac 720
   aatgagacac cagggattag atatcagtac aatgtgcttc cacagggatg gaaaggatca 780
   ccagcaatat tccagtgtag catgacaaaa atcttagagc cttttagaaa acaaaatcca 840
   gacatagtca tctatcaata catggatgat ttgtatgtag gatctgactt agaaataggg 900
   cagcatagaa caaaaataga ggaactgaga caacatctgt tgaggtgggg atttaccaca 960
   ccagacaaaa aacatcagaa agaacctcca ttcctttgga tgggttatga actccatcct 1020
   gataaatgga cagtacagcc tatagtgctg ccagaaaagg acagctggac tgtcaatgac 1080
   atacagaaat tagtgggaaa attgaattgg gcaagtcaga tttatgcagg gattaaagta 1140
   aggcaattat gtaaacttct taggggaacc aaagcactaa cagaagtagt accactaaca 1200
   gaagaagcag agctagaact ggcagaaaac agggagattc taaaagaacc ggtacatgga 1260
   gtgtattatg acccatcaaa agacttaata gcagaaatac agaagcaggg gcaaggccaa 1320
   tggacatatc aaatttatca agagccatta aaaatctgaa aacaggaaag tatgcaagaa 1380
   tgaagggtgc cmtaataggt aatag 1405
<210> 36
   <211> 2851
   <212> DNA
   <213> Artificial sequence
<220>
   <223> Description of Artificial sequence: Synthetic HIV-1 protease sequence with a start codon at position -1 and a stop codon after 2511 more nucleic acids
<400> 36
   ggatccttta acatgcctca gatcactctt tggcagcgac ccctcgtcac aataaagata     60
   ggggggcaat taaaggaagc tctattagat acaggagcag atgatacagt attagaagaa     120
   atgaatttgc caggaagatg gaaaccaaaa atgatagggg gaattggagg ttttatcaaa    180
   gtaagacagt atgatcagat actcatagaa atctgcggac ataaagctat aggtacagta    240
   ttagtaggac ctacacctgt caacataatt ggaagaaatc tgttgactca gattggctgc    300
   actttaaatt ttcccattag tcctattgag actgtaccag taaaattaaa gccaggaatg    360
   gatggcccaa aagttaaaca atggccattg acagaagaaa aaataaaagc attagtcgaa    420
   atttgtacag aaatggaaaa ggaaggaaaa atttcaaaaa ttgggcctga aaatccatac    480
   aatactccag tatttgccat aaagaaaaaa gacagtacta aatggagaaa attagtagat    540
   ttcagagaac ttaataagag aactcaagat ttctgggaag ttcaattagg aataccacat    600
   cctgcagggt taaaacagaa aaaatcagta acagtactgg atgtgggcga tgcatatttt    660
   tcagttccct tagataaaga cttcaggaag tatactgcat ttaccatacc tagtataaac    720
   aatgagacac cagggattag atatcagtac aatgtgcttc cacagggatg gaaaggatca    780
   ccagcaatat tccagtgtag catgacaaaa atcttagagc cttttagaaa acaaaatcca    840
   gacatagtca tctatcaata catggatgat ttgtatgtag gatctgactt agaaataggg    900
   cagcatagaa caaaaataga ggaactgaga caacatctgt tgaggtgggg atttaccaca    960
   ccagacaaaa aacatcagaa agaacctcca ttcctttgga tgggttatga actccatcct   1020
   gataaatgga cagtacagcc tatagtgctg ccagaaaagg acagctggac tgtcaatgac   1080
   atacagaaat tagtgggaaa attgaattgg gcaagtcaga tttatgcagg gattaaagta   1140
   aggcaattat gtaaacttct taggggaacc aaagcactaa cagaagtagt accactaaca      1200
   gaagaagcag agctagaact ggcagaaaac agggagattc taaaagaacc ggtacatgga      1260
   gtgtattatg acccatcaaa agacttaata gcagaaatac agaagcaggg gcaaggccaa      1320
   tggacatatc aaatttatca agagccattt aaaaatctga aaacaggaaa gtatgcaaga      1380
   atgaagggtg cccacactaa tgatgtgaaa caattaacag aggcagtaca aaaaatagcc      1440
   acagaaagca tagtaatatg gggaaagact cctaaattta aattacccat acaaaaggaa      1500
   acatgggaag catggtggac agagtattgg caagccacct ggattcctga gtgggagttt      1560
   gtcaataccc ctcccttagt gaagttatgg taccagttag agaaagaacc cataatagga      1620
   gcagaaactt tctatgtaga tggggcagcc aatagggaaa ctaaattagg aaaagcagga      1680
   tatgtaactg acagaggaag acaaaaagtt gtccccctaa cggacacaac aaatcagaag      1740
   actgagttac aagcaattca tctagctttg caggattcgg gattagaagt aaacatagtg      1800
   acagactcac aatatgcatt gggaatcatt caagcacaac cagataagag tgaatcagag      1860
   ttagtcagtc aaataataga gcagttaata aaaaaggaaa aagtctacct ggcatgggta      1920
   ccagcacaca aaggaattgg aggaaatgaa caagtagata aattggtcag tgctggaatc      1980
   aggaaagtac tatttttaga tggaatagat aaggcccaag aagaacatga gaaatatcac      2040
   agtaattgga gagcaatggc tagtgatttt aacctaccac ctgtagtagc aaaagaaata      2100
   gtagccagct gtgataaatg tcagctaaaa ggggaagcca tgcatggaca agtagactgt      2160
   agcccaggaa tatggcagct agattgtaca catttagaag gaaaagttat cttggtagca      2220
   gttcatgtag ccagtggata tatagaagca gaagtaattc cagcagagac agggcaagaa      2280
   acagcatact tcctcttaaa attagcagga agatggccag taaaaacagt acatacagac      2340
   aatggcagca atttcaccag tactacagtt aaggccgcct gttggtgggc ggggatcaag      2400
   caggaatttg gcattcccta caatccccaa agtcaaggag taatagaatc tatgaataaa      2460
   gaattaaaga aaattatagg acaggtaaga gatcaggctg aacatcttaa gacagcagta      2520
   caaatggcag tattcatcca caattttaaa agaaaagggg ggattggggg gtacagtgca      2580
   ggggaaagaa tagtagacat aatagcaaca gacatacaaa ctaaagaatt acaaaaacaa      2640
   attacaaaaa ttcaaaattt tcgggtttat tacagggaca gcagagatcc agtttggaaa      2700
   ggaccagcaa agctcctctg gaaaggtgaa ggggcagtag taatacaaga taatagtgac      2760
   ataaaagtag tgccaagaag aaaagcaaag atcatcaggg attatggaaa acagatggca      2820
   ggtgagattg tgggcaagta gacaggatta g                                     2851
<210> 37
   <211> 2876
   <212> DNA
   <213> Artificial Sequence
<220>
   <223> Description of Artificial Sequence: Synthetic HIV-1 protease sequence with a start codon at position -1 and a stop codon after 2544 more nucleic acids
<400> 37
   ggatccttta acatgcctca gatcactctt tggcagcgac ccctcgtcac aataaagata      60
   ggggggcaat taaaggaagc tctattagat acaggagcag atgatacagt attagaagaa     120
   atgaatttgc caggaagatg gaaaccaaaa atgatagggg gaattggagg ttttatcaaa    180
   gtaagacagt atgatcagat actcatagaa atctgcggac ataaagctat aggtacagta    240
   ttagtaggac ctacacctgt caacataatt ggaagaaatc tgttgactca gattggctgc    300
   actttaaatt ttcccattag tcctattgag actgtaccag taaaattaaa gccaggaatg    360
   gatggcccaa aagttaaaca atggccattg acagaagaaa aaataaaagc attagtagaa    420
   atttgtacag aaatggaaaa ggaaggaaaa atttcaaaaa ttgggcctga aaatccatac    480
   aatactccag tatttgccat aaagaaaaaa gacagtacta aatggagaaa attagtagat    540
   ttcagagaac ttaataagag aactcaagat ttctgggaag ttcaattagg aataccacat    600
   cctgcagggt taaaacagaa aaaatcagta acagtactgg atgtgggcga tgcatatttt    660
   tcagttccct tagataaaga cttcaggaag tatactgcat ttaccatacc tagtataaac    720
   aatgagacac cagggattag atatcagtac aatgtgcttc cacagggatg gaaaggatca    780
   ccagcaatat tccagtgtag catgacaaaa atcttagagc cttttagaaa acaaaatcca    840
   gacatagtca tctatcaata catggatgat ttgtatgtag gatctgactt agaaataggg    900
   cagcatagaa caaaaataga ggaactgaga caacatctgt tgaggtgggg atttaccaca    960
   ccagacaaaa aacatcagaa agaacctcca ttcctttgga tgggttatga actccatcct   1020
   gataaatgga cagtacagcc tatagtgctg ccagaaaagg acagctggac tgtcaatgac   1080
   atacagaaat tagtgggaaa attgaattgg gcaagtcaga tttatgcagg gattaaagta   1140
   aggcaattat gtaaacttct taggggaacc aaagcactaa cagaagtagt accactaaca   1200
   gaagaagcag agctagaact ggcagaaaac agggagattc taaaagaacc ggtacatgga   1260
   gtgtattatg acccatcaaa agacttaata gcagaaatac agaagcaggg gcaaggccaa   1320
   tggacatatc aaatttatca agagccattt aaaaatctga aaacaggaaa atatgcaaga   1380
   atgaagggtg cccacactaa tgatgtgaaa caattaacag aggcagtaca aaaaatagcc   1440
   acagaaagca tagtaatatg gggaaagact cctaaattta aattacccat acaaaaggaa   1500
   acatgggaag catggtggac agagtattgg caagccacct ggattcctga gtgggagttt   1560
   gtcaataccc ctcccttagt gaagttatgg taccagttag agaaagaacc cataatagga   1620
   gcagaaactt tctatgtaga tggggcagcc aatagggaaa ctaaattagg aaaagcagga   1680
   tatgtaactg acagaggaag acaaaaagtt gtccccctaa cggacacaac aaatcagaag   1740
   actgagttac aagcaattca tctagctttg caggattcgg gattagaagt aaacatagtg   1800
   acagactcac aatatgcatt gggaatcatt caagcacaac cagataagag tgaatcagag   1860
   ttagtcagtc aaataataga gcagttaata aaaaaggaaa aagtctacct ggcatgggta   1920
   ccagcacaca aaggaattgg aggaaatgaa caagtagatg ggttggtcag tgctggaatc   1980
   aggaaagtac tatttttaga tggaatagat aaggcccaag aagaacatga gaaatatcac 2040
   agtaattgga gagcaatggc tagtgatttt aacctaccac ctgtagtagc aaaagaaata 2100
   gtagccagct gtgataaatg tcagctaaaa ggggaagcca tgcatggaca agtagactgt 2160
   agcccaggaa tatggcagct agattgtaca catttagaag gaaaagttat cttggtagca 2220
   gttcatgtag ccagtggata tatagaagca gaagtaattc cagcagagac agggcaagaa 2280
   acagcatact tcctcttaaa attagcagga agatggccag taaaaacagt acatacagac 2340
   aatggcagca atttcaccag tactacagtt aaggccgcct gttggtgggc ggggatcaag 2400
   caggaatttg gcattcccta caatccccaa agtcaaggag taatagaatc tatgaataaa 2460
   gaattaaaga aaattatagg acaggtaaga gatcaggctg aacatcttaa gacagcagta 2520
   caaatggcag tattcatcca caattttaaa agaaaagggg ggattggggg gtacagtgca 2580
   ggggaaagaa tagtagacat aatagcaaca gacatacaaa ctaaagaatt acaaaaacaa 2640
   attacaaaaa ttcaaaattt tcgggtttat tacagggaca gcagagatcc agtttggaaa 2700
   ggaccagcaa agctcctctg gaaaggtgaa ggggcagtag taatacaaga taatagtgac 2760
   ataaaagtag tgccaagaag aaaagcaaag atcatcaggg attatggaaa acagatggca 2820
   ggtgatgatt gtgtggcaag tagacaggat gaggattaac actcgagaaa gattag 2876
<210> 38
   <211> 33
   <212> DNA
   <213> Artificial Sequence
<220>
   <223> Description of Artificial Sequence: Synthetic FgagPol oligonucleotide
<400> 38
   gcggagtcta gaaggagaga gatgggtgcg aga 33
<210> 39
   <211> 40
   <212> DNA
   <213> Artificial Sequence
<220>
   <223> Description of Artificial sequence: Synthetic RGagPol oligonucleotide
<400> 39
   ctaatctttc tcgagtgtta atcctcatcc tgtctacttg 40
<210> 40
   <211> 18
   <212> DNA
   <213> Artificial Sequence
<220>
   <223> Description of Artificial Sequence: Synthetic 5' Gal primer
<400> 40
   tgaataacca ctttaact 18
<210> 41
   <211> 60
   <212> DNA
   <213> Artificial Sequence
<220>
   <223> Description of Artificial sequence: Synthetic 3' RP-12 primer
<400> 41
   cattcctggc tttaatttta ctggtacagt ctcaataggg ctaatttaaa aatttaaagt 60
<210> 42
   <211> 54
   <212> DNA
   <213> Artificial Sequence
<220>
   <223> Description of Artificial sequence: Synthetic 5' F3 primer
<400> 42
   tatactttaa cgtcaaggag aaaaaacccc ggatccttta acatgcctca gatc 54
<210> 43
   <211> 18
   <212> DNA
   <213> Artificial Sequence
<220>
   <223> Description of Artificial sequence: Synthetic 3' M13F primer
<400> 43
   gttttcccag tcaccacg 18
<210> 44
   <211> 53
   <212> DNA
   <213> Artificial Sequence
<220>
   <223> Description of Artificial Sequence: Synthetic sense Gal-gp-rh sequence
<400> 44
   tatactttaa cgtcaaggag aaaaaacccc ggatccatgt acgatgtacg atg 53
<210> 45
   <211> 53
   <212> DNA
   <213> Artificial Sequence
<220>
   <223> Description of Artificial sequence: Synthetic antisense Gal-gp-rh sequence
<400> 45
   atatgaaatt gcagttcctc ttttttgggg cctaggtaca tgctacatgc tac 53
<210> 46
   <211> 54
   <212> DNA
   <213> Artificial Sequence
<220>
   <223> Description of Artificial Sequence: synthetic sense M13-gp-rh sequence
<400> 46
   taataggtaa taggtaagag ctccaattcg ccctatagtg agtcgtatta caat 54
<210> 47
   <211> 54
   <212> DNA
   <213> Artificial sequence
<220>
   <223> Description of Artificial sequence: Synthetic antisense M13-gp-rh sequence
<400> 47
   attatccatt atccattctc gaggttaagc gggatatcac tcagcataat gtta 54
<210> 48
   <211> 33
   <212> DNA
   <213> Artificial sequence
<220>
   <223> Description of Artificial sequence: synthetic F1071-120B primer
<400> 48
   gagggatcca tgtggggtag agacaacaac tcc 33
<210> 49
   <211> 19
   <212> DNA
   <213> Artificial sequence
<220>
   <223> Description of Artificial sequence: Synthetic 5'-a435 primer
<400> 49
   caggctaatt ttttaaggg 19
<210> 50
   <211> 19
   <212> DNA
   <213> Artificial Sequence
<220>
   <223> Description of Artificial sequence: Synthetic R-a435 primer
<400> 50
   cccttaaaaa attagcctg 19
<210> 51
   <211> 60
   <212> DNA
   <213> Artificial sequence
<220>
   <223> Description of Artificial Sequence: Synthetic F1071B1 primer
<400> 51
   ggatccatga tgacagcatg tcagggagtg ggaggacccg gccataaggc aagagttttg 60
<210> 52
   <211> 22
   <212> DNA
   <213> Artificial Sequence
<220>
   <223> Description of Artificial Sequence: Synthetic 5' GP7 primer
<400> 52
   atgatgacag catgtgaggg ag 22
<210> 53
   <211> 20
   <212> DNA
   <213> Artificial Sequence
<220>
   <223> Description of Artificial Sequence: Synthetic 3' R772 primer
<400> 53
   cctgaaaatc catayaayac 20
<210> 54
   <211> 59
   <212> DNA
   <213> Artificial Sequence
<220>
   <223> Description of Artificial Sequence: Synthetic 5' F-pc-rh primer
<400> 54
   atactttaac gtcaaggaga aaaaaccccg gatccatgtg gggtagagac aacaactcc 59
<210> 55
   <211> 53
   <212> DNA
   <213> Artificial sequence
<220>
   <223> Description of Artificial Sequence: Synthetic R2139S1 primer
<400> 55
   gagctcttaa tccattcctg gctttaattt tactggtaca gtttcaattg gac 53
<210> 56
   <211> 56
   <212> DNA
   <213> Artificial Sequence
<220>
   <223> Description of Artificial Sequence: Synthetic sequence for optimum homologous recombination and for the expression of the viral protease
<400> 56
   ggatccctat tgagactgta ccagtaaaat taaagccagg aatggattaa gagctc 56
<210> 57
   <211> 88
   <212> DNA
   <213> Artificial Sequence
<220>
   <223> Description of Artificial Sequence: synthetic modified nucleic acid fragments for optimum homologous recombination and for the expression of the viral protease
<400> 57
   ggatccatgt ggggtagaga caacaactcc ctcgagtcct attgagactg taccagtaaa 60
   attaaagcca ggaatggatt aagagctc 88
<210> 58
   <211> 60
   <212> DNA
   <213> Artificial Sequence
<220>
   <223> Description of Artificial Sequence: synthetic 5' F-pc-rh primer
<400> 58
   tatactttaa cgtcaaggag aaaaaacccc ggatccatgt ggggtagaga caacaactcc 60

## Description

### RELATED APPLICATIONS

This application claims the priority of U.S. applications Ser. No. 12/503,174 and Ser. No. 12/504,456 filed Jul. 15, 2009 and Jul. 16, 2009 .

### TECHNICAL FIELD

This disclosure relates to methods for determining the sensitivity or resistance of retroviruses isolates to molecules, therapeutic retroviral treatments based on viral protease inhibitors, and diagnostic kits derived from the implementation of the methods.

### BACKGROUND

The etiological agents of AIDS are the human immunodeficiency viruses types 1 and 2. These viruses, which share certain clinical and biological characteristics, have major differences, in particular with regard to the ways in which the host is infected. Thus, infection by HIV-2 is more difficult than by HIV-1 (Ancelle R, 0 Bletry, A C Baglin, F Brun-Vezinet, M A Rey and P Godeau, 1987, Long incubation period for HIV-2 infection. Lancet. 1:688-9; Marlink R, P Kandki, I Thior, K Travers, G Eisen, T Siby, I Traore, C C Hsieh, M C Dia and E H Gueye. 1994. Reduced rate of disease development after HIV-2 infection as compared to HIV-1. Science. 265:1587-90; Adjorlolo-Johnson G, K M De Cock, E Ekpini, K M Vetter, T Sibailly, K Brattegaard, D Yavo, R Doorly, J P Whitaker and L Kestens. 1994. Prospective comparison of mother-to-child transmission of HIV-1 and HIV-2 in Abidjan, Ivory Coast. JAMA. 272:462-6; Marlink, R. 1996. Lessons from the second AIDS virus, HIV-2. AIDS. 10:689-99.), the plasma viral load of individuals infected by HIV-2 is less high and/or lower than that in individuals infected by HIV-1 (Andersson S, H Norrgren, Z da Silva, A Biague, S Bamba, S Kwok, C Christopherson, G Biberfeld, and J Albert. 2000. Plasma viral load in HIV-1 and HIV-2 singly and dually infected individuals in Guinea-Bissau, West Africa: significantly lower plasma virus set point in HIV-2 infection than in HIV-1 infection. Arch. Intern. Med. 160:3286-93; Popper S J, A D Sarr, K U Travers, A Gueye-Ndiaye, S Mboup, M E Essex, and P J Kanki. 1999. Lower human immunodeficiency virus (HIV) type 2 viral load reflects the difference in pathogenicity of HIV-1 and HIV-2. J Infect Dis. 180:1116-21.), and the individuals infected by HIV-2 develop the illness more slowly (Vittinghoff E, S Scheer, P O'Malley, G Colfax, S D Holmberg and S P Buchbinder. 1999. Combination antiretroviral therapy and recent declines in AIDS incidence and mortality. J Infect Dis. 179:717-20; Blanco R, Carrasco, L, and Ventoso, I. 2003. Cell killing by HIV-1 protease. J. Biol. Chem. 278:1086-93; Liu H, Krizek J, and Bretscher A. 1992. Construction of a GAL1-regulated yeast cDNA expression library and its application to the identification of genes whose overexpression causes lethality in yeast. Genetics 132:665-673).

HIV-2 was identified for the first time in West Africa in 1986 (Clavel F, D Guetard, F Brun-Vezinet, S Chamaret, M A Rey, M 0 Santos-Ferreira, A G Laurent, C Dauguet, C Katlama, and C Rouzioux. 1986. Isolation of a new human retrovirus from West African patients with AIDS. Science. 233:343-6). In this region, the prevalence of HIV-2 varies between 1% and 10% (Langley C L, E Benga-De, C W Critchlow, I Ndoye, M D Mbengue-Ly, J Kuypers, G Woto-Gaye, S Mboup, C Bergeron, K K Holmes, and N B Kiviat. 1996. HIV-1, HIV-2, human papillomavirus infection and cervical neoplasia in high-risk African women. AIDS. 10:413-7; Poulsen A G, B Kvinesdal, P Aaby, K Molbak, K Frederiksen, F Dias and E Lauritzen. 1989. Prevalence of and mortality from human immunodeficiency virus type 2 in Bissau, West Africa. Lancet. 1:827-31; Wilkins A, D Ricard, J Todd, H Whittle, F Dias, and A Paulo Da Silva 1993. The epidemiology of HIV infection in a rural area of Guinea-Bissau. AIDS. 7:1119-22). The majority of these cases of infection by HIV-2, outside West Africa, are found in European countries and especially in Portugal where the individuals infected by HIV-2 represent 13% of the population infected by human immunodeficiency viruses (Soriano V, P Gomes, W Heneine, A Holguin, M Doruana, R Antunes, K Mansinho, W M Switzer, C Araujo, V Shanmugam, H Lourenco, J Gonzalez-Lahoz and F Antunes. 2000. Human immunodeficiency virus type 2 (HIV-2) in Portugal: clinical spectrum, circulating subtypes, virus isolation, and plasma viral load. J. Med. Virol. 61:111-6). In France, it has been estimated that 1% of the population infected by HIV is infected by the type 2 virus.

In developed countries, the individuals infected by HIV-1 and/or by HIV-2 are treated by chemical therapy, composed of molecules having an inhibiting activity for one or other of the two viral enzymes: Reverse Transcriptase and Protease.

Moreover, the individuals infected by HIV-1 and/or by HIV-2 are also treated by chemical therapy, composed of molecules having an inhibiting activity for the viral entry process or for the viral enzymes: Reverse Transcriptase, Protease, and Integrase.

Although that treatment has significantly helped to reduce morbidity and mortality caused by HIV infection (Palella F J, Jr, K M Delaney, A C Moorman, M O Loveless, J Fuhrer, G A Satten, D J Aschman and S D Holmberg. 1998. Declining morbidity and mortality among patients with advanced human immunodeficiency virus infection. HIV Outpatient Study Investigators. N. Engl. J. Med. 338:853-60; Vittinghoff E, S Scheer, P O'Malley, G Colfax, S D Holmberg and S P Buchbinder. 1999. Combination antiretroviral therapy and recent declines in AIDs incidence and mortality. J. Infect. Dis. 179:717-20), some cases of therapeutic failure have been observed.

The possibility of amplifying, from the plasma, RNA or cell DNA of the individuals infected by HIV-1 and in therapeutic failure, has made it possible to understand at the molecular level the spontaneous or progressive inefficacy of therapeutic treatments. The determination, in particular, of the nucleic sequence of the two viral enzymes Reverse Transcriptase and Protease has shown the appearance of a certain number of mutations. The results obtained during studies in vitro in which wild type viral strain (and therefore sensitive to treatments) carried the mutations have clearly demonstrated the implication of these mutations in the resistance of the virus to treatment.

Researchers have therefore done a certain amount of work on these mutations and the resistances that they generate to orient and guide the choice of therapeutic treatment and optimize its efficacy.

Two different technical approaches were developed to orient therapeutic treatments. One consisted of searching only the already known mutations within the nucleic acid sequences coding for the viral proteins and is called the genotyping approach. The other one, that does not need knowledge of the presence of resistant mutations within the viral sequences, consists of testing in a cell based system the inhibition of viral replication in the presence of inhibiting molecules, and is called the phenotyping approach.

Unfortunately, the economic strategies of the laboratories have the majority of the time led to a general lack of interest in the scientific community with regard to the treatment of patients infected by HIV-2 (the populations most affected by HIV-2 being mainly those in developing countries) or have led to unsuitable solutions: treatments, tests and analyses that are too expensive, diagnoses, for example, phenotyping diagnoses that are too lengthy or impossible to implement on site, absence of competent structures in the country concerned, etc.

Thus, the results obtained during the various studies carried out on HIV-2 have not been sufficiently consistent to make it possible to formulate a correlation between a particular mutation of the HIV-2 protease, and a resistance phenotype.

It should be noted that, the progression of the illness being slower in individuals infected by HIV-2 than in those infected by HIV-1, the counting of T CD4 cells and determination of the plasma viral load do not rapidly take account of the emergence of resistant strains in patients under treatment.

There exist at the present time several companies that provide the resistance profile of an HIV strain isolated from an infected patient through phenotyping. Conceptionally the three tests resemble each other and are based on the ability of each protease inhibitor to inhibit the release of an infectious recombinant virus comprising the protease of the virus infecting the patient. The companies are: EUROFINS-Viralliance (France), which produces Phenoscript.TM., Virco-Johnson & Johnson (Belgium-USA), which produces Antivirogram.TM., and Monogram Biosciences, ex Virologic (United States), which produces Phenosense.TM.. In the three cases, performing those tests requires significant logistic organization, personnel skilled in molecular biology and virology, and expensive infrastructures of the P3 secure laboratory type (it would appear that a complete profile would currently cost between 800 and 1,000 euros per sample). The delay existing between the time when the biological material arrives at the laboratories and the time when the resistance profile is established varies, for each strain of HIV-1, between two and three weeks.

Under these circumstances, putting on the market a reliable rapid test, for example, a phenotyping test that is simple to implement and inexpensive has become imperative. Such a test would assist treating physicians to monitor the appearance of resistant strains in patients infected by retroviruses, in particular HIV 1 or 2, in particular for deprived populations. Moreover, this test could also be used for a "high speed" and/or "high throughput" research for new molecules having inhibiting activity for the retrovirus protease.

M'Barek Najoua Ben et al, 2006 (Retrovirology, 6 September 2006, vol. 3, no.1) discloses a functional test for determining the resistance or sensitivity of an HIV-2 protease to protease inhibitors, based on cytotoxicity involved by the expression of the HIV-2 mature protease in said yeast, wherein said cytotoxicity is abolished by protease inhibitors.

It is known that the expression of the HIV-1 protease in the presence of a short upstream sequence of the protease domain of the Gag-Pol precursor results in processing of active protease in E. coli, whereas the expression of a wild type protease extended only with the initial methionine does not permit to recover proteolytic activity (Wan Min et al, Biochemical Journal, vol. 316, no. 2, 1996, pages 569-573).

### SUMMARY

We provide a method of determining sensitivity or resistance of isolates of HIV retroviruses to a molecule including a) amplifying sequences coding for a protease of a retrovirus to be studied, with or without the or some of amino acid sequences situated upstream and downstream of a cleavage site of a precursor in which the amino acid sequences are situated, b) recombining fragments of DNA, a final product of the amplification, and an expression vector allowing expression of sequence coding for the protease of the retrovirus to be studied under control of a known inducible promoter through co-transformation of the vector and the DNA fragments with at least one yeast cell, c) culturing co-transformed yeast cell or cells to obtain a sufficient number of transformants to perform a sensitivity or resistance test, and recovering transformants issuing from the co-transformed cell, on any suitable medium, d) incubating the transformants in the presence of a molecule to be tested, e) qualitatively or quantitatively analyzing the living cells, and f) deducing the sensitivity or resistance phenotype.

We also provide a diagnostic kit that performs the method, including nucleotide primers selected from the group consisting of SEQ ID NO 38, 39, 40, 41, 42, 48, 51, 52, 53, 54, 55, 56, 57 and 58; at least one expression vector; at least one strain of yeast; and at least one multi-well plate or other support.

### BRIEF DESCRIPTION OF THE DRAWINGS

FIGS. 1 and 2 are schematic representations of the co-transformation of the pRS316-Gal1/10M vector cleaved by the NotI restriction enzyme with the product of the second PCR to obtain a transformed yeast cell having the sequence of the HIV-2 protease under the control of the inducible promoter Gall.

FIG. 3 is a schematic representation of the counting of living and dead cells and a determination of the phenotype.

FIG. 4 is a schematic representation of a diagnostic kit.

FIG. 5a shows a pair of graphs of percentage of living cells as a function of inhibitor concentration.

FIG. 5b shows photos of cell growth as a function of resistance to inhibitor.

### DETAILED DESCRIPTION

We provide methods for determining the sensitivity or resistance of retroviruses such as HIV to molecules, therapeutic treatments based on viral protease inhibitors, by the use of yeast. In other words, we provide for the use of yeast to determine the resistance or sensitivity of the viral protease to molecules, and/or the chemical molecules used in the context of therapeutic protocols. We also provide diagnostic kits comprising the elements necessary for implementing the method.

More particularly, the methods make it possible to determine, quickly and at low cost, the resistance phenotype of the HIV-2 protease in infected patients. The methods also make it possible to determine, quickly and at low cost, the resistance phenotype of HIV-1 protease in infected patients. The methods further allow the "high speed" and/or "high throughput" search for new chemical molecules having an inhibiting activity for the viral protease with a view to developing novel therapeutic treatments.

We thus provide means permitting the rapid and low-cost definition of the resistance phenotype of the HIV-2 or HIV-1 protease in infected patients, by virtue of the use of yeast. Our methods can also be implemented to define the resistance phenotype of the HIV-1 protease, or the protease of any other retrovirus.

The sequences coding some therapeutic targets, for example, reverse transcriptase protease, and integrase, as well as the so-called "structure proteins" (matrix, capsid, nucleocapsid), are situated within a common polypeptide precursor called Gag-Pol, coded by the gag-pol viral gene (Clavel F, Guyader M, Guetard D, Salle M, Montagnier L. Alizon M. 1986. Molecular cloning and polymorphism of the human immune deficiency virus type 2. Nature, 324:691-5). It is the action of the viral protease that, by hydrolysis of specific peptide bonds referred to as cleavage sites, framing the primary sequences of the various constituents of the precursor, is responsible for the release of these proteins (Oroszlan S and Luftig RB. 1990. Retroviral proteinases. Curr Top Microbiol Immunol. 157:153-85). It has been shown that, for the HIV-1 protease, the amino acid sequences situated upstream and downstream of the cleavage site fulfill an important role in the recognition event of the enzyme for its substrate, and therefore are determinant for its proteolytic activity (Pettit S C, Simsic J, Loeb D D, Everitt L, Hutchison CA 3rd, Swanstrom R. 1991. Analysis of retroviral protease cleavage sites reveals two types of cleavage sits and the structural requirements of the P1 amino acid. J. Biol. Chem. 266:14539-47, Pettit S C, Moody M D, Wehbie R S, Kaplan A H, Nantermet P V, Klein C A, Swanstrom R. 1994). The p2 domain of human immunodeficiency virus type 1 Gag regulates sequential proteolytic processing and is required to produce fully infectious virions (J Virol. 68:8017-27, Moody M D, Pettit S C, Shao W, Everitt L, Loeb D D, Hutchison C A 3rd, Swanstrom R. 1995). A side chain at position 48 of the human immunodeficiency virus type-1 protease flap provides an additional specificity determinant (Virology. 207:475-85, Boross P, Bagossi P, Copeland T D, Oroszlan S, Louis J M, Tozser J. 1999. Effect of substrate residues on the P2' preference of retroviral proteinases. Eur J Biochem. 264:921-9).

A scientific article that appeared in 2003 demonstrated that the expression of the HIV-1 protease by the yeast Saccharomyces cerevisiae caused the death of the latter through a still unknown mechanism, the consequence of which was the cell lysis of the yeast in question (Blanco R, Carrasco L and Ventoso I. 2003. Cell killing by HIV-1 protease. J. Biol. Chem. 278:1086-93).

We demonstrated that the same phenomenon occurred when the yeasts expressed the HIV-2 protease. Consequently, by inhibiting viral enzymatic activity by modification of its catalytic site, we succeeded in preventing the appearance of this cell event.

In addition, Blanco et al (Blanco R, Carrasco L, and Ventoso I. 2003. Cell killing by HIV-1 protease. J. Biol. Chem. 278:1086-93) also showed that the inhibition of the HIV-1 protease by one of the inhibitors used in anti-HIV therapy inhibited cell death of the yeast caused by expression of the viral protease. Because of this fact, it is possible to quantitatively measure the sensitivity and resistance of the protease of infected individuals to the various inhibited molecules.

Moreover, we worked on the smallest active Gag-Pol precursor that, once expressed in yeast, induces cell death through protease activity. Since the definition of a functional protease precursor protein is the one from which the protease can, sequentially, be cleaved off and kill then the expressing yeast, we created and expressed a large number of truncated Gag-Pol precursors in yeast where the protease cleavage sit was either present or absent by specific nucleic acid mutation. We surprisingly found that the smallest active protease precursor was defined as the smallest Gag-Pol sequence, containing the protease flanked by its cleavage sits that only induces yeast cell death when the cleavage site situated at the N-terminal part of the protease sequence is present. In the absence of this one, the expressed precursor is unable to disturb cell growth.

We have surprisingly found that a sequence encoding for the protease preceded by the 6 amino acids of the cleavage sit and followed by at least one amino acid of the other cleavage site was the smallest active precursor form that induces cell death when expressed in yeast.

Our methods therefore make it possible to determine, in the cellular context of the yeast, the sensitivity phenotype of the viral protease of a retrovirus such as HIV-2 or HIV-1, to drugs with an inhibiting activity.

In other words, it makes it possible to determine the sensitivity or resistance of isolates of retroviruses such as HIV (human immunodeficiency virus) to chemical molecules having an inhibiting activity on the viral protease or to therapeutic treatments based on inhibitors of the viral protease, characterized by the use for this purpose of at least one yeast whose cell lysis is caused by expression of the retrovirus protease.

We surprisingly discovered that, not only the specific sequence coding for protease, but also a precursor sequence of HIV-1 protease when incorporated in an expression vector, through a co-transformation proceeding in yeast allows the expression of protease and cause the death of the yeast. In other words, we discovered that sequences comprising protease HIV-1 coding sequence extracted from for example, infected blood or cells are functionally expressed in yeast.

Therefore, our methods allow us to determine the sensitivity or resistance of retrovirus protease to molecules in a cell based non-infectious system in the presence of its natural substrate. Further, our methods allow us to test molecules having an effect on the activity of the protease and also on expression of the protease. Moreover, our methods allow us to test molecules having a direct effect on the activity of the protease toward its natural substrate that is, for example, the protease precursor. For example, the method allows us to test molecules inhibiting the activity of protease by acting on the sequence coding for the protease, and/or coding for the protease precursor, by acting on the translation of the protease, by acting on the transcription mechanism, and/or on the protease activity.

Moreover, our methods allow for the determination of the sensitivity or resistance of protease having at least one mutation, for example, in the protease and the protease precursor coding nucleic acids sequence.

Schematically, the methods comprise an expression vector, choice of the cell system, method of expressing proteases of infected individuals and test of susceptibility to drugs.

It comprises the following steps:

optionally extracting the nucleic acids, DNA and/or RNA from body fluids or cells (blood or other) taken from an individual or animal infected by the retrovirus, by any suitable means;

amplifying the sequences coding for the protease of the retrovirus to be studied;

recombining the fragments of DNA, the final product of the amplification, and an expression vector allowing the expression of the sequence coding for the protease, and/or any form or length of the protease precursor of the retrovirus to be studied under the control of a known inducible promoter, through co-transformation of the vector and DNA fragments with at least one yeast cell whose cell lysis is caused by the expression of the retrovirus protease;

culturing the co-transformed yeast cell or cells to obtain a sufficient number of transformants to perform the sensitivity or resistance test, and recovery of the transformants issuing from the co-transformed cell, on any suitable medium;

incubating the transformants in the presence of, preferably with an increasing concentration, of each molecule to be tested;

qualitatively or quantitatively analyzing the living cells; and

deducing the resistance phenotype.

The nucleic acids may be extracted from cells infected by a retrovirus, and/or body fluids from an infected individual or animal, and/or blood from an infected individual or animal, and/or from infected culture cell media.

The molecules to be tested, also called "test molecules," are selected from the group comprising molecules of a library, chemical molecules, natural molecules and molecules extracted from plants.

The test molecules may be selected from the group comprising chemical molecules having an inhibiting activity on the viral protease, therapeutic treatments based on inhibitors of the viral protease or of the viral maturation.

The sequences of the protease amplified according to the method include those coding for the isolated protein or those coding for the protein and comprising all or some of the amino acid sequences situated upstream and downstream of the cleavage site of the protein precursor in which they are situated.

The retrovirus may be HIV-1.

The amplified sequences coding for the protease of the retrovirus to be studied according to the method can be mutated or non-mutated sequence. The amplified sequences coding for the protease of the retrovirus, for example, HIV-1, to be studied may be selected from the group comprising SEQ ID N° 10 to 37.

The yeast used in the method may be of the type of *Saccharomyces cerevisiae.*

The nucleic acids may be amplified, for example, by Polymerase Chain Reaction using couple of primers selected from the group comprising SEQ ID N° 38, 39, 40, 41, 42, 48, 51 to 58.

The protocols used in the laboratory with a view to obtaining yeast transformants coding for an exogenous gene generally begin with a first step for obtaining the fragments of DNA coding for the gene of interest, either by gene amplification (Polymerase Chain Reaction (PCR) technique), or by release of the gene by virtue of the action of the restriction enzymes, which cuts the DNA containing the sequence of interest. This first step is equivalent in time to half a day's work.

The DNA fragment released is then sub-cloned in an expression vector by the action of the DNA Ligase enzyme (an operation lasting one night) and the product of the reaction is amplified in a bacterium, after its transformation (one day to obtain bacteria having incorporated plasmid DNA, and one and a half days of obtaining and characterizing the transform ant sought, containing the plasmid coding for the gene of interest).

Moreover, to produce sufficient quantities of the plasmid containing the gene of interest with a view to transformation of the yeast, the bacterial clone obtained in the previous step may be amplified (one night) and the plasmids purified by conventional known methods (one day).

The purified plasmid obtained is then used to transform the selected yeast strain (1/2 day).

The transformed yeast strain is obtained approximately 4 days after the transformation event.

Consequently, by using the conventional protocols well known to persons skilled in the art, it is possible to obtain a sufficient quantity of yeast transformants for a subsequent study of a gene of interest, for example, for developing a sensitivity or resistance test, the time that elapses between the preparation of the DNA fragment coding for the gene of interest and the obtaining of the yeast strain expressing it is a minimum of 8 days. Moreover, as disclosed above, several techniques are used.

The magnitude of these delays and the multiplicity of the techniques used necessarily give rise to high production costs, incompatible with the development of a rapid sensitivity or resistance test that is simple to implement and inexpensive.

In other words, the prior art methods of obtaining of yeast transformants are undesirable.

The culture of the transformants can be done, for example, in liquid medium and/or solid medium. The medium used in our methods can be any known medium and suitable for the culture of the transformants, for example, minimal synthetic media containing a carbon source such as glucose or galactose and lacking or not a specific nutrient, for example, uracil.

"Qualitative or quantitative analysis" means any analysis of living cells known in the art. For example, it can be a step of counting or scoring the living cells, for example, by measuring the absorbance, for example, at 600 nm of a liquid medium, or by eye counting, by observation when the transformants are cultured, for example, on a solid medium. It can also be, for example, the result of laboratory testing for the sensitivity of the transformants to a test molecule or another method involving the use of a test molecule. For example, it can be a semi-quantitative way based on diffusion (Kirby-Bauer method); small discs containing a test molecule or impregnated paper discs, are dropped in different zones of the culture, for example, onto an agar plate, which is a nutrient-rich environment in which the transformants can grow. The molecule to be tested diffuses in the area surrounding each disc. It can also be, for example, a quantitative way based on dilution: a dilution series of molecule to be tested is established, that is, for example, a series of reaction vials with progressively lower concentrations of molecule substance.

The sensitivity or resistance of protease can be deduced, for example, by measuring the half maximal inhibitory concentration (IC50), by comparing the living number of cells between transformants incubated with molecules and non-incubated with molecules.

Considering the studies on the ability of yeast to repair "broken DNA" (nicked DNA) by the homologous recombination mechanism, we found that, during this cell event, a DNA molecule is repaired at a precise point in its sequence, by putting in place homologous sequences at the "nicking" site and taken within another DNA molecule. By using this physiological phenomenon, it is possible to introduce a defined sequence within the "nicked" DNA provided that the defined sequence is framed on each side by sequences identical to those situated around the "nicking" site.

The minimum size of the homologous sequences present in the two DNA molecules for the recombination event to be able to take place is approximately 30 to 40 pairs of bases.

This technique very advantageously simplifies obtaining the transformants by reducing in particular the number of manipulations which involve a significant reduction in the experimentation time necessary (approximately half compared to known protocols) and in production cost. Use of this technique also enables a large number of samples to be manipulated at the same time.

We provide a detailed description of preferred examples of implementation of our methods below.

### Example 1

This example of implementation is used for preference when the protease expressed is the isolated protein. When the protease is expressed with all or some of the amino acid sequences situated upstream and downstream of the cleavage site of the protein precursor that contains it, the primers and nucleotide fragments disclosed above will be modified accordingly.

### 1--Preparation and Modification of the Expression Vector:

The aim of the modification is to be able sub-clone the gene of the protease from virus infecting each infected individual studied and transform the yeast with the protease gene obtained, by a simple and rapid procedure (a single-step procedure).

We created a modified version of the vector pRS316-Gal1/10, which comprises the inducible promoter GAL1/10 in position 5' of the cloning site of the gene to be expressed (Liu H, Krizek J, and Bretscher A. 1992. Construction of a GAL-1 regulated yeast cDNA expression library and its application to the identification of genes whose overexpression causes lethality in yeast. Genetics 132:665-673).

Because of this, the viral gene is expressed when the cell is transformed with this vector in the presence of Galactose and the gene is not expressed when the cells transformed are in the presence of glucose as a carbon source.

For the fragment of amplified viral DNA to be able to be inserted by homologous recombination in the expression vector, the vector must be modified by adding to it, just after the sequence of the inducible promoter, a primer 5' of approximately 40 pairs of bases, followed by a single restriction site (to be able to linearize the vector), and a primer 3' of approximately 40 pairs of bases. Although this modified and linearized vector is a good substrate for the homologous recombination event, the sequence introduced at position 5' (between the gene and the promoter) must not inhibit the transcription of the genes.

To produce the modified version of the expression vector pRS316-Gal1/10, we exchanged the BamHI-Sac1 fragment of this site with another DNA fragment also framed by the BarnH1-Sac1 restriction sites that contain (from 5' to 3'):
- the BamH1 restriction site, unique in the vector, followed by
- the 35 to 45 nucleotides in the HIV-2 sequence situated just upstream of the protease, followed by
- an NotI restriction site, unique in the vector, followed by
- the 35 to 45 nucleotides of the HIV-2 sequence situated just downstream of the protease, followed by
- the Sac1 restriction site, unique in the vector.

Modifications to the size of this fragment of approximately 80-90 pairs of bases were carried out to optimize experimental cloning, transformation and expression system. A single modified fragment among 10 different ones that were tested was sufficient for the homologous recombination and expression of the viral protease.

The sequence of this fragment is as follows: The pRS316-Gal1/10 expression vector thus modified is called pRS316-Gal 1/10M.

### 2--Choice of the Cell System

Any strain of yeast with auxotrophy markers necessary to allow the selection of the transformant expressing the viral protease, and preferably any strain of *Saccharomyces cerevisiae.*

### 3--Sub-Cloning of the Proteases of Individuals Affected by the Retrovirus, and Transformation of Yeasts in a Single Step

The DNA sequence coding the HIV-2 protease is amplified by the PCR technique, twice.

The first amplification produces a significant quantity of DNA. This reaction takes place using DNA extracted from lymphocytes of the peripheral blood of infected individuals. The nucleotide primers used are of the type:
(SEQ ID N° 2) Sense primer: 5'-GAAAGAAGCCCCGCAACTTC-3'
(SEQ ID N° 3) Antisense primer: 5'-GGGATCCATGTCACTTGCCA-3'

The second amplification frames the protease of an initialization codon of the transcription (ATG) and of a termination codon of the transcription (TAA) and adds on each side the sequences approximately 40 nucleotides that we brought to the vector when it was modified. This PCR reaction takes place on the product of the first PCR with the primers of the type:

Framing the protease by the initiation and termination codons is that of the isolated protease or the protein comprising all or some of the amino acid sequences situated upstream and downstream of the cleavage site of the precursor in which it is situated.

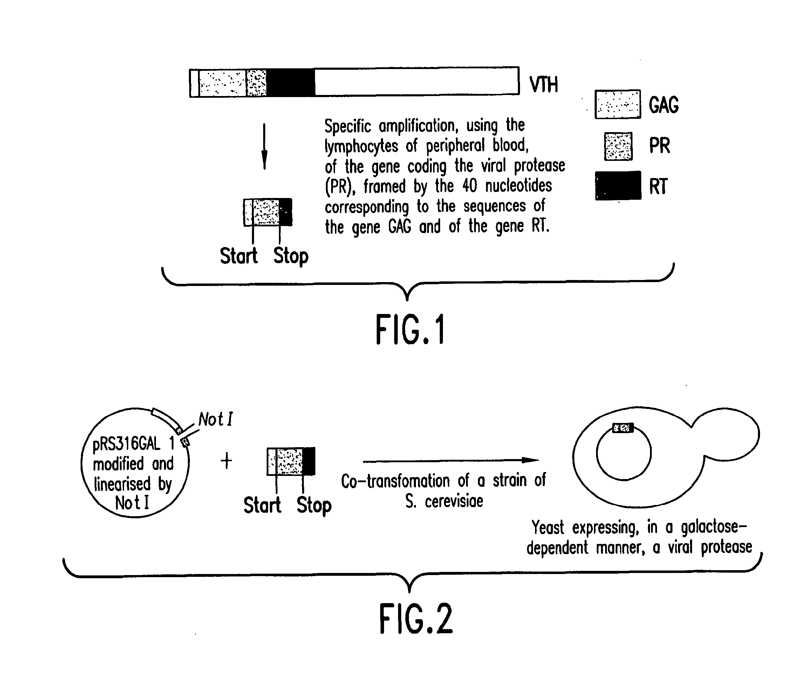

Co-transformation of the pRS316-Gal1/10M vector cleaved by the NotI restriction enzyme with the product of the second PCR makes it possible, through the homogeneous recombination event that took place in the cell, to obtain a transformed yeast cell carrying the sequence of the HIV-2 protease under the control of the inducible promoter Gal (FIGS. 1 and 2).

### 4--The Test

A sample of peripheral blood is taken from an individual infected with HIV-2. The lymphocytes issuing from this sampling are purified or not, and their DNA extracted by known methods.

The DNA undergoes the two aforementioned PCR reactions to create the fragment of DNA, carrying the sequence of the protease with or without the or some of the amino-acid sequences situated upstream and downstream of the cleavage site of the precursor in which it is situated and compatible with the expression vector to cause the phenomenon of homogeneous recombination in the transformed cells.

After purification of the product of the second PCR, a yeast strain having a genotype ura3 is co-transformed with the pRS316Gal1/10M linear vector (by its NotI site).

The transformants potentially producing the protease are recovered on any suitable carrier such as, for example, gelose composed of agar, glucose as a source of carbon, and a synthetic environment with a deficiency of uracil.

Approximately 10⁵ cells, issuing from a single transformant, are deposited and distributed in 12 wells with a 96-well plate and incubated in galactose in the presence of 11 increasing concentrations of each inhibitor to be tested. The twelfth well does not contain any inhibitor.

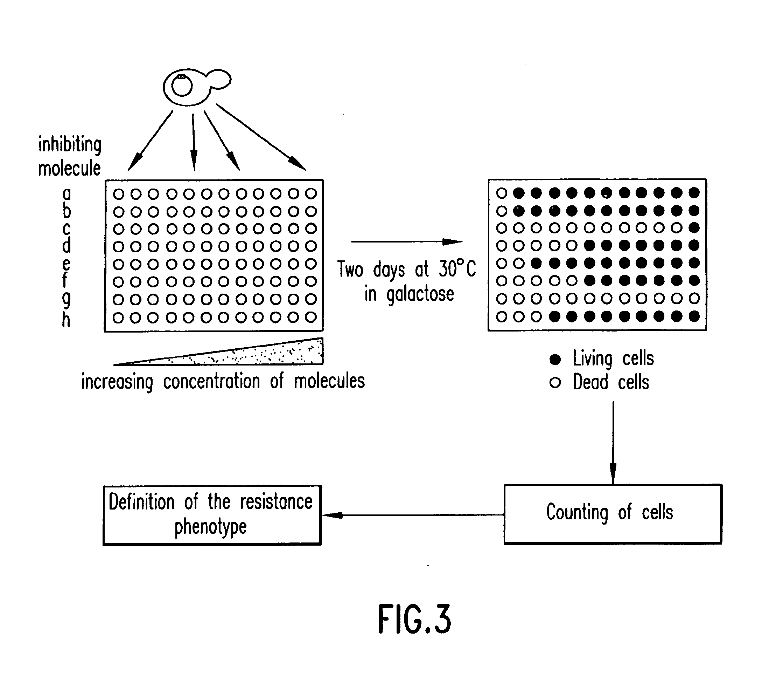

After 36-48 hours at 30.degree. C., the living cells are counted by a densitometric reading at 600 nm (FIG. 3).

The dose necessary to inhibit half the cell growth under these conditions, compared with the cell growth in the absence of drugs and in the presence of glucose (IC⁵⁰), defines the susceptibility or resistance of this specific strain.

The test was also done on plasma issuing from this sampling, purified or not, and their RNA or DNA extracted by known methods. In this case, these nucleic acids undergo the aforementioned RT-PCR and PCR reactions, if they are of RNA type, and no RT phase if they are of type DNA.

The interval of time between the blood sampling and the definition of the resistance profile is only one week.

### Example 2

In this example, the expression vector was modified as follows:

A first modified version of the vector pRS316-Gal1/10 was created by exchanging the BamH1-Sac1 fragment of this vector with a DNA fragment, also framed by the BamH1-Sac1 restriction sites that contains (from 5' to 3'):
- the BamH1 restriction site, unique in the vector, followed by
- around 40 nucleotides coding for amino acids situated after the HIV-1 protease coding sequence, followed by
- a stop codon, followed by [0102]a Sac1 restriction site, unique in the vector.

Modifications of the nucleotide sequences coding for amino acids situated after the protease coding sequence were carried out for the experimental cloning, transformation and expression system. Among several modified fragments created and tested for selected homologous recombination and expression of a precursor form of the viral protease, that consists of the protease coding sequence followed by 13 amino acids, one example includes:

A second modified version of the vector pRS316-Gal1/10 was created by exchanging the BamH1-Sac1 fragment of this vector with a DNA fragment, also framed by the BamH1-Sac1 restriction sites that contains (from 5' to 3'):
- the BamH1 restriction site, unique in the vector, followed by
- a start codon, followed by
- around 20 nucleotides coding for amino acids situated before the HIV-1 protease coding sequence, followed by
- a XhoI restriction site, unique in the vector, followed by
- around 40 nucleotides coding for amino acids situated after the HIV-1 protease coding sequence, followed by
- a stop codon, followed by
- a Sac 1 restriction site, unique in the vector.

Modifications of the nucleotide sequences coding for amino acids situated before and after the protease coding sequence were carried out for the experimental cloning, transformation and expression system. Among several modified fragments created and tested for selected homologous recombination and expression of a precursor form of the viral protease, in this case the short precursor in the type of SEQ ID N° 15, one example includes:

### 2--Choice of the Cell System

Any strain of yeast with necessary auxotrophy markers to allow the selection of the transformant expressing the viral protease, and preferably any strain of *Saccharomyces cerevisiae.* In this example *Saccharomyces cerevisiae* was used.

### 3--Sub-Cloning of the Proteases of Individuals Affected by the Retrovirus, and Transformation of Yeasts in a Single Step

The nucleic acid sequence coding the HIV protease, or any of its precursor forms, were amplified by the RT-PCR technique, followed by a PCR reaction as in Example 1.

The nucleotide primers used for the RT-PCR reaction were of the type:
(SEQ ID N° 52) Primer5'GP7: ATGATGACAGCATGTGAGGGAG, and
(SEQ ID N° 53) Primer3'R772: CCTGAAAATCCATAYAAYAC.

The second PCR reaction takes place on the product of the first PCR with either the primers of the type: and or the primers of the type:
(SEQ ID N° 55) Primer R2139S1:,
and

In one case, the co-transformation of the pRS316-Gal1/10M-3 vector cleaved by the BamH1 restriction enzyme with the product of the second PCR, performed with primers SEQ ID N° 42 and 55, makes it possible, through the homologous recombination event that took place in the cell, to obtain a transformed yeast cell, carrying the sequence of a precursor form of the HIV-1 protease that consists of the protease coding sequence followed by 13 amino acids, under the control of the inducible promoter Gal.

In the other case, co-transformation of the pRS316-Gal1/10M4 vector cleaved by the XhoI restriction enzyme with the product of the second PCR, performed with primers SEQ ID NO 54 and 55, makes it possible, through the homologous recombination event that took place in the cell, to obtain a transformed yeast cell, carrying the sequence of a precursor form of the HIV-1 protease (like SEQ ID N° 15) under the control of the inducible promoter Gal.

### 4--The Test

A sample of peripheral blood is taken from an individual infected with HIV-1. The plasma or lymphocytes issuing from this sampling are purified or not, and their RNA or DNA extracted by known extraction kits, like Q1Aamp Viral RNA Mini Kit from QIAGEN (The Netherlands).

These viral nucleic acids undergo the same treatments as defined in Example 1, to amplify the DNA sequence coding for the protease precursor.

After purification of the product of the second PCR, a yeast strain having a genotype ura3 is either co-transformed with the pRS316Gal1/10M-3 linear vector (by its BamH1 site) or co-transformed with the pRS316Gal1/10M-4 linear vector (by its XhoI site).

The transformants potentially producing the protease are recovered on any suitable carrier such as gelose composed of agar, glucose as a source of carbon, and a synthetic environment with a deficiency of uracil.

Approximately 10⁵ cells, issuing from a single transformant, are deposited and distributed in 12 wells with a 96-well plate and incubated either in liquid or solid galactose containing media in the presence of at least 8 increasing concentrations of each inhibitor to be tested. One well does not contain any inhibitor.

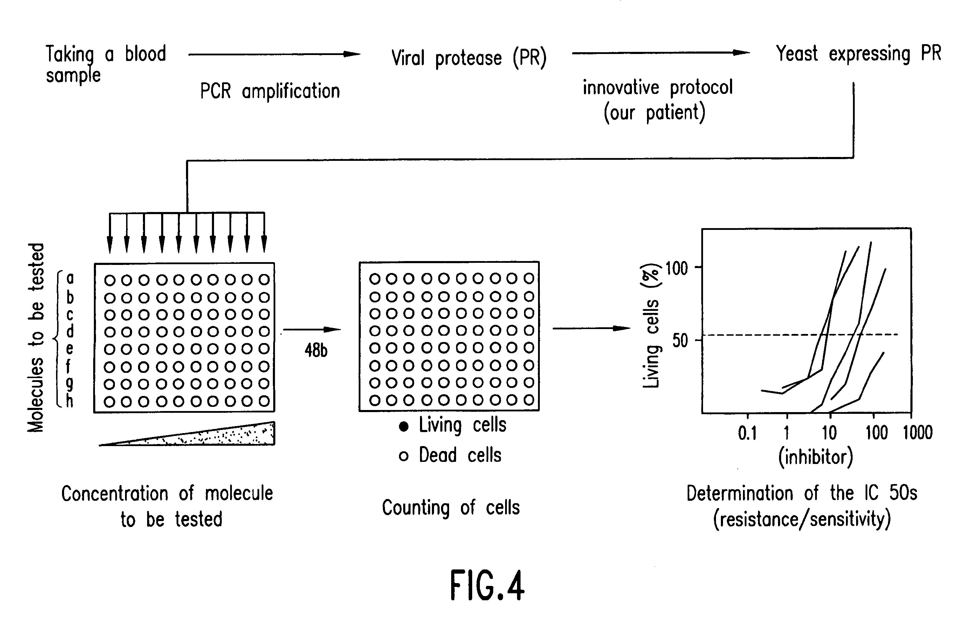

After 36 to 48 hours at 30.degree. C., the living cells are counted by a densitometric reading at 600 nm (FIG. 4) when the test was performed in liquid media or by eye when the test was performed in solid media.

The dose necessary for inhibiting half the cell growth under these conditions, compared with the cell growth in the absence of drugs and in the presence of glucose (IC⁵⁰), defines the susceptibility or resistance of this specific strain.

The interval of time between blood sampling and definition of the resistance profile is only one week.

### Example 3

### Example of Protease Sequences Amplifications

### 1--Integral Protease Precursor Sequence

The integral protease precursor sequence was amplified by PCR from the laboratory viral pNL4.3 DNA strain with using the following primers:
(SEQ ID N° 38) 5' Primer F-GagPol GCGGAGTCTAGAAGGAGAGAGATGGGTGCG AGA, and
(SEQ ID N° 39)
   3' Primer R-GagPol CTAATCTTTCTCGAGTGTTAATCCTCATCCTG TCTACTTG.

The used PCR program was as follows:

3 min at 95.degree. C., followed by 35 cycles of 1 min at 95.degree. C., 30 seconds at 55.degree. C., 2 min at 72.degree. C., and finished with one round of 7 min at 72.degree. C.

The PCR product (SEQ ID N° 18) was purified by standard procedures digested by restriction enzyme XbaI (Invitrogen, USA), following the manufacturing recommendations and introduced, through overnight DNA ligation (T4 DNA Ligase from Gibco USA), as manufacturing recommendations into previously XbaI digested pRS316-Gal/10 vector.

### 2--Protease Precursor Sequence Starting at the Beginning of the Protease Coding Sequence and Ending at the End of the Pol Coding Region (SEQ N° 37)

The PCR reaction was performed on purified DNA construct in pRS316-Gal/10 vector obtained in Item 1. PCR conditions and other experiences were the same as conditions described in previous Item 1.

The protease precursor sequence from protease coding sequence until the end of the gag-pol coding sequence was obtained using the following primers: and
(SEQ ID N° 43) 3' Primer M13F: GTTTTCCCAGTCACCACG for the amplification step.

The primers were selected to provide sequences situated on both sides of the cloning site in the expression vector.

Co-transformation of linear pRS316-Gal1/10 vector (digested with BamH1) with PCR product produced, by a recombination event in the cell, a transformed yeast carrying the precursor sequence under the control of inducible Gal promoter.

### 3--Different Size of Protease Precursor Sequence

In this example the construct obtained in previous Item 2 was used.

Plasmid containing the gag-pol precursor sequences starting at the protease and ending at the end of the pol region was linearized by digestion with restriction enzyme XhoI (Invitrogen, USA) according to manufacturing recommendations.

After, 6 micrograms of the linearized plasmid were digested with 1 unit of Bal31 exonuclease enzyme (BioLabs, USA) at 30.degree. C. following the manufacturing recommendations. Equal aliquots were taken every 20 minutes during 2 hours incubation, and the reaction was stopped with 25 mM EDTA. This produced a DNA partial digest and fragments of different size. These fragments, present in each Bal31 digestion aliquot, were ligated to the following double strand oligonucleotides in a ratio of 50 pmoles oligonucleotide to 1 microgram DNA:

These oligonucleotides further contain initiation and termination codons and identical sequences to the pRS316-Gal1/10 vector.

The co-transformation of linearized pRS316-Gal1/10 vector by BamH1-Sac1 enzymes with the ligation product previously digested with Kpnl restriction enzyme (Invitrogen, USA), following the recommendation of manufacturer produce, by homologous recombination done in cell, a library of transformed yeasts carrying each one C terminal deleted forms of the gag-pol precursor starting at the protease sequence under the control of inducible promoter Gal.

### 4--A Truncated Protease Precursor Sequence Comprising a Single Mutation (Addition of a Single A Nucleotide) to Mimic the Natural Open Reading Frame Shift of the Gag-Pol Gene Transcription

The PCR conditions and other experiments were the same as conditions described in previous Item 1.

A mutated precursor sequence containing an additional A nucleotide at position 1638 was made. The primers used for the PCR reaction were:
(SEQ ID N° 49) primer 5'-a435: CAGGCTAATTTTTTAAGGG,
(SEQ ID N° 50) primer R-a435: CCCTTAAAAAATTAGCCTG,
and

The mutated precursor was made in two steps of PCR. In a first PCR, 2 independent reactions, using as template the construct obtained in previous Item 2, involved pairs of primers, SEQ ID N° 49 and SEQ ID N° 55 for one and SEQ ID N° 50 and SEQ ID N° 51 for the second, to introduce a further A (Adenine) at position 1638 of the sequence.

These tFwo PCR products were mixed and diluted to be a template for the second PCR which was realized with primers SEQ ID NO 51 and SEQ ID NO 55. The newly obtained DNA fragment was digested with BamH1 and Sac 1 restriction enzymes (Invitrogen, USA and Fermentas, Canada, respectively), following recommendations of the manufacturer, and ligated with T4 DNA Ligase, (Gibso, USA) following recommendations of the manufacturer, in pRS316-Gal1/10 vector previously digested with BamH1 and Sac1 restriction enzymes (Invitrogen, USA and Fermentas, Canada, respectively), following recommendations of the manufacturer.

### 5--Cloning a Precursor Sequence of HIV-1 Protease from Infected Patient

An HIV-1 protease precursor form coding sequence was amplified from purified total RNA extracted from blood plasma of infected patients by RT-PCR followed by a PCR reaction, as described in Example 2.

The primers used for the RT-PCR reaction were:
(SEQ ID N° 52) Primer 5' GP7: ATGATGACAGCATGTGAGGGAG, and
(SEQ ID N° 53) Primer 3' R772: CCTGAAAATCCATAYAAYAC.

The second amplification frames the protease between an initialization codon of the transcription (ATG) and a termination codon of the transcription (TAA). ATG was situated 70 nucleotides above of the first codon of the protease. TAA was situated 150 nucleotides below of the last codon. Moreover, the second amplification adds on both sides sequences corresponding to the vector to clone it in yeast by homologous recombination. This PCR was made on the first PCR product with the following primers: and

### Example 4

### Example of Determining the Resistance/Sensitive Character of a Protease from HIV-1 Infected Individual

An HIV-1 short protease precursor form, of the size of SEQ ID N° 15, coding sequence was amplified by RT-PCR followed by a PCR reaction, as described in Example 2, from purified total RNA extracted from blood plasma of an infected patient, namely P#10, known to carry HIV-1 strains resistant to protease inhibitors like lopinavir, saquinavir and darunavir.

The followed procedure was as described in Example 3 (5), using the following primers:
(SEQ ID N° 52) Primer 5' GP7: ATGATGACAGCATGTGAGGGAG, and
(SEQ ID N° 53) Primer 3' R772: CCTGAAAATCCATAYAAYAC, for the RT-PCR step, and the primers: and

The obtained final PCR product was cloned into pRS316-Gal1/10M-4 by the co-transforming single step procedure described in Example 2 (3). The transformed yeast cell was of the strain BY4741 (Euroscarf, Germany) having the following genotype: MATa, his3.Δ.1, leu2.Δ.0, met15.Δ.0, ura3.Δ.0.

To define the ability of lopinavir, saquinavir and darunavir molecules to inhibit the cloned protease, hence the resistance pattern, the procedure described in Example 3 (5) was performed to obtain yeast cells expressing sensitive and resistant proteases. The sensitive or resistant character of the cloned protease was determined in liquid and in solid media.

When the resistance test was performed in liquid media, the higher concentration of the molecules tested were:
50 microMolar for lopinavir,
100 microMolar for darunavir,
400 microMolar for saquinavir,
[7 serial dilutions, one to one, of those solutions were present in the test.

In parallel, the same procedure was followed to test the ability of the same protease inhibitors to act on the short protease precursor form (SEQ ID N° 15) expressed in BY4741 yeast transformants. In this case, the DNA amplification was performed, on purified pNL4.3 plasmid carrying the total sensitive strain HIV-1 genome, by a PCR reaction using the following primers: and

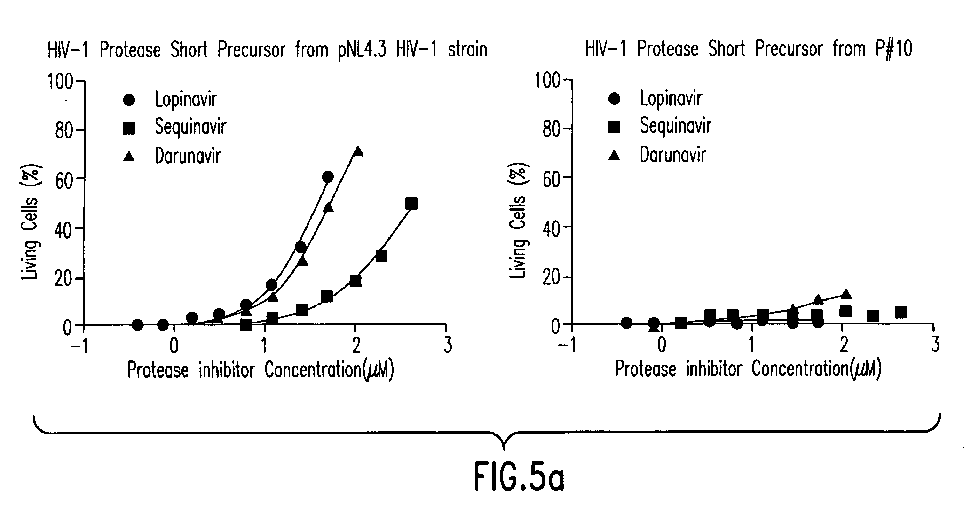

The obtained results, presented as the percentage of living cells as a function of the inhibitor concentration in FIG. 5a, show:

i) lack of inhibition, by the three inhibiting molecules tested, of the viral protease in its precursor context amplified from patient P#10,

ii) inhibition, by the three inhibiting molecules tested, of a sensitive viral protease in its precursor context amplified from a wild HIV-1 strain, namely pNL4.3.

The data was processed by the software GraphPad Prism v5.0 (GraphPad Inc, USA) to obtain the corresponding IC50 values that are showed in Table 1:

**TABLE 1**

| Protease Inhibitor | IC50 (microMolar) | |
|---|---|---|
| | PNL4.3 protease | P#10 protease |
| Lopinavir | 39.2 | >10⁵ |
| Darunavir | 50.5 | 1907.0 |
| Saquinavir | 433.2 | >10⁵ |

Therefore, the viral protease from patient P#10 is resistant to the tested inhibitor molecules.

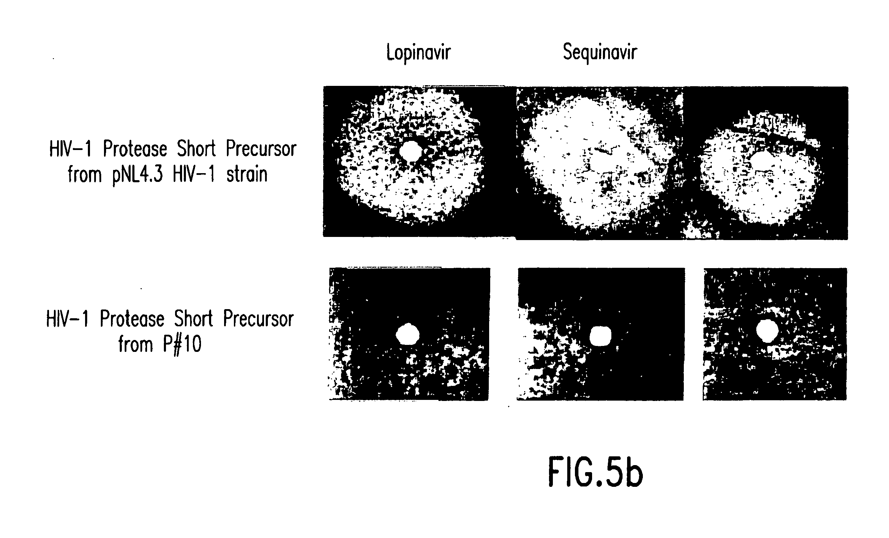

When the resistance test was performed in solid media, the yeast transformant expressing each viral protease was plated on synthetic minimal media, agar plate, in the presence of 2% Galactose. A 5 mm diameter paper disk (Minitrans-Blot, Bio-Rad USA) impregnated with 10 microliters of either 0.5 milliMolar lopinavir, 0.5 m milliMolar darunavir, or 1 milliMolar saquinavir, was placed in the center of the plate. After 3 days at 30.degree. C. observation by naked eye was performed. When the expressed viral protease was sensitive to the inhibitor, a halo of growing cells was observed, whereas no growing cells were observed when the viral protease expressed in transformed yeasts were resistant to inhibitor (FIG. 5b).

As demonstrated in this example, the method allows determination of the sensitivity or resistance of isolate of HIV retrovirus to molecules.

### Example 5

### Diagnostic Kit for Resistance to Retroviruses

A diagnostic kit also determines the sensitivity or resistance of retrovirus isolates to therapeutic retroviral treatments based on inhibitors of the viral protease. It may comprise:

nucleotide primers and, for example, nucleotide primers as previously disclosed for implementation of the amplification of DNA coding for the retrovirus protease by the PCR technique, namely: for the first amplification primers of the type:
(SEQ ID N° 2) Sense primer: 5'-GAAAGAAGCCCCGCAACTTC-3'
(SEQ ID N° 3) Antisense primer: 5'GGGATCCATGTCACTTGCCA-3'
for the second amplification, primers of the type:
(SEQ ID N° 5) Antisense primer: 5'GCGGAGCTCGCTTTAGCATTATTTTTA TTGGCTCTACTGCGGCCGCTTAA GATT3' and/or primers selected from the group comprising SEQ ID N° 38, 39, 40, 41, 42, 48, 51 to 58;
at least one expression vector and, for example, the plasmid modified and linearized according to our methods and as previously described, for example, the plasmid pRS316-Gal1/10M, M-3, M-4;
at least one strain of yeast with the necessary auxotrophy marker to permit selection of the transformant expressing the viral protease, and preferably a strain of *Saccharomyces cerevisiae;* and
at least one multi-well plate or any other suitable support.

Naturally, when amplification is carried out on the RNA or DNA coding for the protease of the retrovirus with all or some of the amino acid sequences situated upstream and downstream of the cleavage site of the protein precursor that contains it, the primers and nucleotide fragments will be modified accordingly.

A principle of the kit, also illustrated in FIG. 4, is as follows:

Using the blood of the patient, the gene coding for the viral protease is amplified by an RT-PCR reaction with the primers described in the method and supplied in the kit.

Amplification of the gene of the viral protease using cell DNA is also possible.

The amplified product and the modified and linearized vector (also supplied in the kit) are used for transforming a strain of yeast, also supplied in the kit, in a multi-well plate.

In a preferred, though in no way limiting fashion, after incubation at 30.degree. in a dry oven in a selective medium containing glucose, 2 microliters of the cell suspension are transferred to a new plate, supplied in the kit, containing increasing concentrations of each active principle used in therapy. 300 microliters of the selected medium containing galactose are added to each well, before incubating at to 30.degree. C. in a dry oven for 36-48 hours.

At the end of incubation, for example, if the support was liquid media, the living cells are counted by a densitometric reading at 600 nm. The dose necessary for inhibiting half the cell growth, compared with the cell growth of the strain of yeast not expressing the viral protease, defines the IC⁵⁰ for each inhibitor. A comparison between the IC.sub.50s obtained and those of a reference wild protease makes it possible to determine any resistance phenotype.

At the end of incubation, for example, if the support is solid as in Example 4, naked eye observation defines the HIV protease sensitive/resistant character towards a molecule, for example, a specific inhibitor of the viral protease from the infected individual, by merely determining whether there is growth (sensitive) or no growth (resistant) of the transformed yeast.

The interval of time between the taking of blood and the definition of the resistance profile is only one week.

## Claims

1. A method of determining sensitivity or resistance of isolates of HIV-1 retrovirus to a molecule comprising:
a) amplifying sequences coding for a protease of a HIV-1 retrovirus to be studied preceded by the 6 amino acids of the cleavage site and followed by at least one amino acid of the other cleavage site, , wherein said cleavage sites corresponds to the specific peptide bonds framing the primary sequences of the HIV-1 protease in the Gag-Pol precursor that are hydrolyzed for the release of said protease,
b) recombining fragments of DNA, a final product of the amplification, and an expression vector allowing expression of sequence coding for the protease of the HIV-1 retrovirus to be studied preceded by the 6 amino acids of the cleavage site and followed by at least one amino acid of the other cleavage site under control of a known inducible promoter through co-transformation of the vector and the DNA fragments with at least one yeast cell,
c) culturing co-transformed yeast cell or cells to obtain a sufficient number of transformants to perform a sensitivity or resistance test, and recovering transformants issuing from the co-transformed cell, on any suitable medium,
d) incubating the transformants in the presence of a molecule to be tested,
e) qualitatively or quantitatively analyzing the living cells, and
f) deducing the sensitivity or resistance phenotype.

2. The method according to of claim 1, wherein said method allow to test molecules having a direct effect on the activity of the protease toward its natural substrate that is the HIV-1 protease precursor.

3. The method according to any one of claims 1 to 2, wherein a mutated HIV-1 precursor sequence containing an additional A nucleotide at position 1638 was made to mimic the natural Open Reading Frame Shift of the Gag-Pol Gene transcription.

4. The method according to any one of claims 1 to 3 comprising before step a), extracting nucleic acids from i) cells infected by a HIV-1 retrovirus and/or ii) body fluids from infected animals, and/or iii) blood from infected animals, and/or iv) infected culture cell media.

5. The method according to claim 4, wherein the nucleic acid is DNA and/or RNA.

6. The method according to claim 4, wherein the nucleic acids are extracted from cells taken from an individual or an animal infected by a HIV-1 retrovirus and/or ii) body fluids from infected animals, and/or iii) blood from infected animals, and/or iv) infected culture cell media.

7. The method according to claim 1, wherein the molecules are at least one selected from the group consisting of molecules of a library, chemical molecules, natural molecules and molecules extracted from plants.

8. The method according to claim 1, wherein the molecules are at least one selected from the group consisting of chemical molecules having an inhibiting activity on the viral protease, therapeutic treatments based on inhibitors of the viral protease.

9. The method according to claim 1, wherein the incubation is performed with an increasing concentration of molecules.

10. The method according to claim 1, wherein said nucleic acid is amplified with a pair primers selected from the group consisting of SEQ ID N° 38, 39, 40, 41, 42, 48, 51 to 58.

11. The method according to claim 1, wherein said nucleic acid is any one of SEQ ID N° 9 to SEQ ID N° 37.

12. The method according to claim 1, wherein the expression vector is plasmid pRS316-Gal 1/10.

13. The method according to claim 11, wherein the expression vector is plasmid pRS316-Gal1/10 comprising inducible promoter Gal1/10 in position 5' of a cloning site of a gene to be expressed, and in which a fragment BamH1-Sac1 is replaced by another fragment of DNA consisting of (from 5' to 3'):
the BamH1 restriction site, unique in the vector, followed by about 20 nucleotides in a retrovirus sequence situated just upstream of the protease, followed by a restriction site, unique in the vector, followed by 0 to 2511 nucleotides of the retrovirus sequence situated downstream of the protease, followed by the Sac1 restriction site, unique in the vector.

14. The method according to claim 13, wherein the about 20 nucleotides in the HIV-1 retrovirus sequence are situated upstream of the protease, followed by an Xhol restriction site.

15. The method according to claim 1, wherein the yeast is Saccharomyces cerevisiae.

16. The method according to claim 1, wherein the culture medium of the co-transformed yeast cells is deficient in glucose.

17. The method according to claim 1, wherein incubation of the transformant is done in galactose.

18. A diagnostic kit that performs the method according to any one of claims 1 to 16, comprising:
nucleotide primers selected from the group consisting of SEQ ID N~ 38, 39, 40, 41, 42, 48, 51, 52, 53, 54, 55, 56, 57 and 58;
at least one expression vector;
at least one strain of yeast; and
at least one multi-well plate or other support.

19. The diagnostic kit according to claim 18, wherein the expression vector is plasmid pRS316-Gal1/10.

20. The diagnostic kit according to claim 18, wherein the yeast is Saccharomyces cerevisiae.

## Patentansprüche

1. Verfahren zur Bestimmung der Empfindlichkeit oder Resistenz von Isolaten des HIV-1-Retrovirus gegenüber einem Molekül, umfassend:
a) Vervielfältigen von Sequenzen, die für eine Protease eines zu untersuchenden HIV-1-Retrovirus codieren, welcher die 6 Aminosäuren der Spaltstelle vorausgehen und auf welche mindestens eine Aminosäure der anderen Spaltstelle folgt, wobei die Spaltstellen den spezifischen Peptidbindungen entsprechen, welche die primären Sequenzen der HIV-1-Protease innerhalb des Gag-Pol-Vorläufers einrahmen, wobei sie zur Freisetzung der Protease hydrolysiert werden,
b) Rekombinieren von DNA-Fragmenten, nämlich eines Endprodukts der Vervielfältigung, mit einem Expressionsvektors, der die Expression einer Sequenz ermöglicht, welche für die Protease des zu untersuchenden HIV-1-Retrovirus codiert, wobei dieser die 6 Aminosäuren der Spaltstelle vorausgehen und dieser mindestens eine Aminosäure der anderen Spaltstelle folgt, und welche der Steuerung durch einen bekannten induzierbaren Promotor unterliegt, indem der Vektor und die DNA-Fragmente eine co-Transformation mit mindestens einer Hefezelle erfahren,
c) Anzüchten der co-transformierten Hefezelle oder -zellen, sodass eine Anzahl an Transformanten erhalten wird, die dafür ausreicht, eine Empfindlichkeits- oder Resistenzprüfung durchzuführen, und Überführen der Transformanten, die aus der co-transformierten Zelle hervorgehen, auf ein beliebiges geeignetes Medium,
d) Bebrüten der Transformanten in Gegenwart eines zu prüfenden Moleküls,
e) qualitative oder quantitative Untersuchung der lebenden Zellen, und
f) Ableiten des Phänotyps hinsichtlich der Empfindlichkeit oder Resistenz.

2. Verfahren nach Anspruch 1, wobei es das Verfahren ermöglicht, Moleküle zu prüfen, die eine direkte Wirkung auf die Aktivität der Protease gegenüber ihrem natürlichen Substrat haben, bei welchem es sich es um den HIV-1-Proteasevorläufer handelt.

3. Verfahren nach einem beliebigen der Ansprüche 1 bis 2, wobei eine mutierte HIV-1-Vorläufersequenz hergestellt wurde, die an der Position 1638 ein zusätzliches Nukleotid A enthält, um die Verschiebung des natürlichen offenen Leserahmens der Gag-Pol-Gentranskription nachzuempfinden.

4. Verfahren nach einem beliebigen der Ansprüche 1 bis 3, wobei es vor dem Schritt a) das Extrahieren von Nukleinsäuren aus i) Zellen, die mit einem HIV-1-Retrovirus infiziert sind, und/oder ii) Körperflüssigkeiten von infizierten Tieren, und/oder iii) Blut von infizierten Tieren, und/oder iv) Medien infizierter Zellkulturen umfasst.

5. Verfahren nach Anspruch 4, wobei es sich bei der Nukleinsäure um DNA und/oder RNA handelt.

6. Verfahren nach Anspruch 4, wobei die Nukleinsäuren aus Zellen extrahiert werden, die i) einem Menschen oder einem Tier entnommen werden, welcher/welches mit einem HIV-1-Retrovirus infiziert ist, und/oder ii) Körperflüssigkeiten von infizierten Tieren, und/oder iii) Blut von infizierten Tieren, und/oder iv) Medien infizierter Zellkulturen.

7. Verfahren nach Anspruch 1, wobei es sich bei den Molekülen um mindestens eines handelt, das aus der Gruppe ausgewählt ist, die aus den Molekülen einer Bibliothek, chemischen Molekülen, natürlichen Molekülen und Molekülen, welche aus Pflanzen extrahiert wurden, besteht.

8. Verfahren nach Anspruch 1, wobei es sich bei den Molekülen um mindestens eines handelt, das aus der Gruppe ausgewählt ist, die aus chemischen Molekülen, welche eine hemmende Wirkung auf die virale Protease haben, therapeutischen Behandlungsmitteln, welche auf Hemmstoffen der viralen Protease beruhen, besteht.

9. Verfahren nach Anspruch 1, wobei die Bebrütung mit einer zunehmenden Konzentration an Molekülen durchgeführt wird.

10. Verfahren nach Anspruch 1, wobei die Nukleinsäure mit einem Paar von Primern vervielfältigt wird, das aus der Gruppe ausgewählt ist, welche aus den SEQ-ID-Nr. 38, 39, 40, 41, 42, 48, 51 bis 58 besteht.

11. Verfahren nach Anspruch 1, wobei es sich bei der Nukleinsäure um eine beliebige der SEQ-ID-Nr. 9 bis SEQ-ID-Nr. 37 handelt.

12. Verfahren nach Anspruch 1, wobei es sich bei dem Expressionsvektor um das Plasmid pRS316-Gal 1/10 handelt.

13. Verfahren nach Anspruch 11, wobei es sich bei dem Expressionsvektor um das Plasmid pRS316-Gal1/10 handelt, welches den induzierbaren Promotor Gal1/10 in 5'-Position einer Klonierungsstelle eines zu exprimierenden Gen umfasst und in welchem ein BamH1-Sac1-Fragment durch ein anderes DNA-Fragment ersetzt ist, das aus Folgendem besteht (von 5' nach 3'):
der BamH1-Restriktionsstelle, welche nur einmal in dem Vektor vorliegt, gefolgt von ungefähr 20 Nukleotiden in einer Retrovirus-Sequenz, die unmittelbar strangaufwärts der Protease gelegen ist, gefolgt von einer Restriktionsstelle, welche nur einmal in dem Vektor vorliegt, gefolgt von 0 bis 2511 Nukleotiden der Retrovirus-Sequenz, die strangabwärts der Protease gelegen ist, gefolgt von der Sac1-Restriktionsstelle, welche nur einmal in dem Vektor vorliegt.

14. Verfahren nach Anspruch 13, wobei die ungefähr 20 Nukleotide in der HIV-1-Retrovirus-Sequenz strangaufwärts der Protease gelegen sind, gefolgt von einer Xhol-Restriktionsstelle.

15. Verfahren nach Anspruch 1, wobei es sich bei der Hefe um *Saccharomyces cerevisiae* handelt.

16. Verfahren nach Anspruch 1, wobei in dem Kulturmedium der co-transformierten Hefezellen ein ungenügendes Glucoseangebot herrscht.

17. Verfahren nach Anspruch 1, wobei die Bebrütung der Transformanten in Galactose erfolgt.

18. Diagnostisches Kit zur Durchführung des Verfahrens nach einem beliebigen der Ansprüche 1 bis 16, umfassend:
Nukleotidprimer, die aus der Gruppe ausgewählt sind, welche aus den SEQ-ID-Nr. 38, 39, 40, 41, 42, 48, 51, 52, 53, 54, 55, 56, 57 und 58 besteht;
mindestens einen Expressionsvektor;
mindestens einen Hefestamm; und
mindestens eine Multi-Kavitätenplatte oder einen sonstigen Träger.

19. Diagnostisches Kit nach Anspruch 18, wobei es sich bei dem Expressionsvektor um das Plasmid p115316-Gal1/10 handelt.

20. Diagnostisches Kit nach Anspruch 18, wobei es sich bei der Hefe um *Saccharomyces cerevisiae* handelt.

## Revendications

1. Une méthode de détermination de la sensibilité ou résistance d'isolats de rétrovirus VIH-1 à une molécule comprenant :
a) l'amplification de séquences codant pour une protéase de rétrovirus VIH-1 à étudier, précédée des six acides aminés du site de clivage et suivie d'au moins un acide aminé de l'autre site de clivage, où lesdits sites de clivage correspondent aux liaisons peptidiques spécifiques encadrant les séquences primaires de la protéase VIH-1 dans le précurseur Gag-Pol qui sont hydrolysées pour la libération de ladite protéase,
b) la recombinaison de fragments d'ADN, un produit final de l'amplification, et d'un vecteur permettant l'expression d'une séquence codant pour la protéase du rétrovirus VIH-1 à étudier, précédée des six acides aminés du site de clivage et suivie d'au moins un acide aminé de l'autre site de clivage, sous le contrôle d'un promoteur inductible connu par cotransformation du vecteur et des fragments d'ADN avec au moins une cellule de levure,
c) la culture de la ou des cellule(s) de levure cotransformée(s) pour obtenir un nombre suffisant de transformants pour l'exécution du test de sensibilité ou résistance, et la récupération des transformants issus de la cellule cotransformée, dans n'importe quel milieu convenable,
d) l'incubation des transformants en présence de la molécule à tester,
e) l'analyse qualitative ou quantitative des cellules vivantes, et
f) la déduction du phénotype de sensibilité ou résistance.

2. La méthode selon la revendication 1, où ladite méthode permet de tester des molécules ayant un effet direct sur l'activité de la protéase sur son substrat naturel qui est le précurseur de la protéase VIH-1.

3. La méthode selon l'une quelconque des revendications 1 à 2, où une séquence de précurseur VIH-1 muté contenant un nucléotide A additionnel en position 1638 est réalisé pour mimer le décalage du cadre de lecture ouvert naturel de la transcription du gène Gag-Pol.

4. La méthode selon l'une quelconque des revendications 1 à 3 comprenant avant l'étape a) l'extraction d'acides nucléiques de i) cellules infectées par un rétrovirus VIH-1 et/ou ii) fluides corporels d'animaux infectés, et/ou iii) sang d'animaux infectés et/ou iv) milieux de culture cellulaire infecté.

5. La méthode selon la revendication 4, où l'acide nucléique est de l'ADN et/ou de l'ARN.

6. La méthode selon la revendication 4, où les acides nucléiques sont extraits de i) cellules obtenues d'un individu ou un animal infecté par un rétrovirus VIH-1 et/ou ii) fluides corporels d'animaux infectés, et/ou iii) sang d'animaux infectés et/ou iv) milieux de culture cellulaire infectés.

7. La méthode selon la revendication 1, où les molécules sont au moins une sélectionnée du groupe consistant en des molécules d'une libraire, des molécules chimiques, des molécules naturelles et des molécules extraites des plantes.

8. La méthode selon la revendication 1, où les molécules sont au moins une sélectionnée du groupe consistant en des molécules chimiques ayant une activité inhibitrice sur la protéase virale, des traitements thérapeutiques basés sur des inhibiteurs de la protéase virale.

9. La méthode selon la revendication 1, où l'incubation est exécutée avec une concentration croissante de molécules.

10. La méthode selon la revendication 1, où ledit acide nucléique est amplifié avec une paire d'amorces sélectionnée à partir du groupe consistant en SEQ ID N°38, 39, 40, 41, 42, 48, 51 à 58.

11. La méthode selon la revendication 1, où ledit aide nucléique est l'un quelconque des SEQ ID NO :9 à SEQ ID NO :37.

12. La méthode selon la revendication 1, où le vecteur d'expression est le plasmide pRS316-Gal1/10.

13. La méthode selon la revendication 11, où le vecteur d'expression est le plasmide pRS316-Gal1/10 comprenant un promoteur inductible Gal1/10 en position 5' d'un site de clonage d'un gène à exprimer, et dans lequel un fragment BamH1-Sac1 est remplacé par un autre fragment d'ADN consistant en (de 5' vers 3') :
le site de restriction BamH1 unique dans le vecteur, suivi d'environ 20 nucléotides dans une séquence de rétrovirus située juste en amont de la protéase, suivie d'un site de restriction, unique dans le vecteur, suivi de 0 à 2511 nucléotides de la séquence de rétrovirus située en aval de la protase, suivis d'un site de restriction Sac1, unique dans le vecteur.

14. La méthode selon la revendication 13, où les environ 20 nucléotides dans la séquence de rétrovirus VIH-1 sont situés en amont de la protéase, suivis d'un site de restriction Xhol.

15. La méthode selon la revendication 1, où la levure est *Saccharomyces cerevisiae.*

16. la méthode selon la revendication 1, où le milieu de culture des cellules de levure cotransformées est déficient en glucose.

17. La méthode selon la revendication 1, où l'incubation du transformant est réalisée dans du galactose.

18. Un kit de diagnostic, qui exécute la méthode selon l'une quelconque des revendications 1 à 16, comprenant :
des amorces nucléotidiques sélectionnées dans le groupe consistante en SEQ ID N°38, 39, 40, 41, 42, 48, 51, 52, 53, 54, 55, 56, 57 et 58 ;
au moins un vecteur d'expression,
au moins une souche de levure, et
au moins une plaque multi-puits ou un autre support.

19. Le kit de diagnostic selon la revendication 18, où le vecteur d'expression est le plasmide pRS316-Gal1/10.

20. Le kit de diagnostic selon la revendication 18, où la levure est *Saccharomyces cerevisiae.*
